# EUROPEAN PATENT SPECIFICATION

(11) **EP 1 545 190 B1**
(45) Date of publication and mention of the grant of the patent: **20.06.2012**
(21) Application number: 03793390.0
(22) Date of filing: 25.08.2003
(51) Int. Cl.: A01H 1/02, C12N 15/31, C12N 15/55, C12N 15/82

(54) **MALE STERILITY RESTORATION AS A SELECTABLE MARKER IN PLANT TRANSFORMATION**
WIEDERHERSTELLUNG DER MÄNNLICHEN STERILITÄT ALS SELEKTIONSMARKER BEI PFLANZENTRANSFORMATION
RESTAURATION DE LA FECONDITE MALE UTILISEE COMME MARQUEUR SELECTIONNABLE DANS LA TRANSFORMATION DE PLANTES

(30) Priority: 23.08.2002 US 405654 P
(43) Date of publication of application: 29.06.2005
(73) Proprietor: BASF Plant Science GmbH, 67056 Ludwigshafen, Rheinland-Pfalz (DE); DNA Landmarks, Inc., St-Jean-sur-Richelieu, Québec J3B 6X3 (CA)
(72) Inventor: LAI, Fang-Ming, Apex, NC 27502 (US); LANDRY, Benoit, S., L'Acadie, Québec J2Y 1B3 (CA)
(74) Representative: Weiss, Wolfgang
(86) International application number: PCT/US2003/026593
(87) International publication number: WO 2004/018639

(56) References cited:
- EP-A- 0 599 042
- WO-A-01/16287
- WO-A-92/05251
- WO-A-97/02737
- WO-A-99/23233
- US-A- 5 013 659
- US-A- 5 658 772
- US-A- 5 929 307
- US-A- 5 981 833
- LUO HONG ET AL: "FLP-mediated recombination for use in hybrid plant production" PLANT JOURNAL, vol. 23, no. 3, August 2000 (2000-08), pages 423-430, XP002301000 ISSN: 0960-7412
- GIDONI DAVID ET AL: "FLP/FRT-mediated restoration of normal phenotypes and clonal sectors formation in rolC transgenic tobacco" TRANSGENIC RESEARCH, vol. 10, no. 4, August 2001 (2001-08), pages 317-328, XP002390415 ISSN: 0962-8819

## Description

### Field of the Invention

This invention relates generally to methods of transforming plants, plant cells, and plant tissue using nucleic acids that encode products which can restore male fertility to a male sterile plant and using the male fertility restoration trait to select for transformed plants comprising a nucleotide sequence of interest.

### Background Art

In plants, the best first filial (F(1)) hybrids have a substantial yield advantage over the best open-pollinated varieties or inbred lines and their parents. This phenomena is termed "heterosis", or hybrid vigor. The observed degree of hybrid vigor varies among species; however, it is typically higher among cross-pollinated species, such as maize and sunflower, and lower among self-pollinated species, such as soybean and wheat. As a result, hybrid varieties of maize largely have replaced open-pollinated varieties because significant improvements in yield, along with other agronomic traits, were realized since the application of hybrid varieties in the 1940's.

To obtain F(1) hybrid seed, it is necessary to cross two inbred parents. Although it is possible to do this via controlled pollinations (i.e. fertilizations) of flower by flower manually, such an approach is labor-intensive and very expensive. Only high-value crops, such as ornamental flowers and the like, could absorb such production costs. In developing improved new Brassica varieties, breeders use self-incompatible (SI), cytoplasmic male sterile (CMS) and nuclear male sterile (NMS) Brassica plants as the female parent (EP 0 853 874 A1). In using these plants, breeders are attempting to improve the efficiency of seed production and the quality of the F1 hybrids and to reduce the breeding costs.

Male sterility has been discovered in many plant species. There are two forms of male sterility, namely cytoplasmic male sterility (CMS), and nuclear male sterility (NMS), which is also referred to as chromosomal or genetic male sterility. Nuclear male sterility genes are normally recessive, and occur in practical all diploid plant species which have been examined, and are present in polyploid plants as well (Frankel et al., 1977, Pollination mechanisms, reproduction and plant breeding, Springer Verlag, 3.4.1.2 and 3.4.2.1; Gottschalk et al., 1983, Monographs on Theoretical and Applied Genetics 7:70). For a diploid plant, such a locus (allele pair) can be homozygous male sterile (ms/ms), homozygous male fertile (Ms/Ms), or heterozygous male fertile (Ms/ms). The phenotype of nuclear male sterility exists only if the plant is homozygous for the recessive nuclear male sterility locus (ms/ms).

In crop plants, such as maize and Brassica species, a convenient way of controlling pollination is achieved by taking advantage of cytoplasmic male sterility ("CMS"). A cytoplasmic male-sterile line, which is incapable of cross- or self-pollination, can be used as a female parent. By crossing such a female parent with a pollen-producing line, fertile hybrid seed can be generated without emasculating the female plants. Therefore, the method of using CMS is efficient in seed production and facility of maintenance of mating parents. The causative factors of CMS have been shown to reside in the cytoplasm of a plant cell, and facilitate formation of ill-formed anthers that do not produce viable pollen (Laughnan et al., 1983, Annual Review of Genetics 17:27-48). Such factors have been determined to be associated with mitochondria, which are maternally inherited.

CMS occurs in many species of plants including maize (Laughnan et al., 1983, Annual Review of Genetics 17:27-48), petunia (Nivison et al., 1989, Plant Cell, 1:1121-1130), the common bean (Janska et al., 1993, Genetics, 135:869-879), *Brassica napus* (Singh et al., 1991, Plant Cell, 3:1349-1362), sunflower (Laver et al., 1991, The Plant Journal, 1:185-193), sorghum (Bailey-Serres et al., 1986, Cell 47(4):567-576), and oats (Mann et al., 1989, Theor. Appl. Genet., 78:293-297). Cytoplasmic male sterility in maize, petunia, beans, and Brassica has been restored to fertility by single dominant nuclear genes.

In maize (Zea mays L.), there are three types of male-sterile cytoplasms: S (USDA), C (Charrua), and T (Texas). These three male-sterile cytoplasms are distinguished by the ability of different nuclear (restorer) genes to restore fertility to the plants with these different cytoplasms (Laughnan et al., 1983, Ann. Rev. Gen. 17:27-48), by mitochondrial DNA restriction endonuclease profiles (Pring et al., 1978), and by 35S-methionine-labeled polypeptides translated in isolated mitochondria (Forde et al., 1978).

When the male-sterile plants with C-, S-, and T-cytoplasms are used as female parents, fertile hybrid plants can be produced only with a male parent carrying the corresponding nuclear restorer genes. These fertility-restorer genes in the nucleus compensate for cytoplasmic dysfunction(s) in the mitochondria that are phenotypically expressed during microsporogenesis and/or microgametogenesis. Plants carrying S- or C-cytoplasm are restored to fertility by a single dominant allele of the rf3 or rf4 locus, respectively. Preliminary results indicate that the rf3 locus maps on chromosome 2L (Kamps et al., 1992, Maize Genet. Coop. Newsl. 66:45), and the rf4 locus maps to chromosome 8 (Sisco, 1991, Crop Sci., 31:1263-1266). In contrast to S- and C-cytoplasms, plants with T-cytoplasm are restored to fertility by the dominant alleles of two loci, rf1 and rf2 (Laughnan et al.,1983, Ann. Rev. Gen. 17:27-48; Levings et al., 1983, Cell. 32(3):659-61), which are located on separate chromosomes. The rf1 locus is localized on chromosome 3, and the rf2 on chromosome 9 (Wise et al., 1994, Theor. Appl. Genet. 88:785-795).

T-cytoplasm is restored to fertility at the sporophytic level; the genetic constitution of the diploid anther tissue, rather than that of the haploid, gametophytic pollen grain. Therefore, a T-cytoplasmic plant, which is heterozygous for both restorer gene loci (Rf1/rf1, Rf2/rf2), will produce all normal pollen even though only one-fourth of the pollen grains carry both Rf1 and Rf2 (Laughnan et al.,1983, Ann. Rev. Gen. 17:27-48). In contrast, S-cytoplasm is restored to fertility at the gametophytic level, and, therefore, an S-cytoplasmic plant, which is heterozygous for rf3 (Rf3/rf3), will produce half normal pollen because only one-half of the pollen grains carry Rf3 (Laughnan et al.,1983, Ann. Rev. Gen. 17:27-48; Schardl et al., 1985, Cell 43(1):361-68).

Since the restorer genes are normally associated with other deleterious traits, use of these restorer genes in the conventional breeding brings unwanted effects in the F(1) progeny. For instance, in T-cytoplasmic maize, CMS is associated with the unique mitochondrial gene T-urf13 (Wise et al., 1987, Proc. Nat'l. Acad. Sci. USA 84:2858-86), and expressed the toxin sensitivity traits (Huang et al., EMBO, 9, 339-247 (1990)). Therefore, it is advantageous to isolate the restorer genes and generate transgenic plants containing the isolated restorer gene as the restorer line for hybrid seed production. US Patent No. 5,981,833 describes cloning of nuclear fertility restorer genes (rf2) in maize and their use in generating plants with these restorer genes for hybrid seed production. The cloned Rf2 gene is highly related to aldehyde dehydrogenase from a variety of species, including Bos taurus (73% homology), Ovis aries (73% homology), horse (74% homology), Aspergillus niger (86% homology), and Homo sapiens (86% homology). It appears that Rf2 encodes a common enzymatic function, aldehyde dehydrogenase, which can be considered a necessary mitochondrial activity, and can also be considered a necessary detoxification step to restore a vital mitochondrial activity.

In Brassica, there are also several forms of CMS, including Anand, Tokumasu's CMS (Paulmann & Robbelen, 1988, Plant Breeding, 100:299-309), and Ogura (Pellan-Delounne et al., 1987, Proc. 7th Int. Rapeseed Conf., Poznan, Poland: 199-203; WO 92/05251) and Polima. Polima, a form of CMS, has been used in the commercial production of F(1) hybrid of rapeseed plants, even though the Polima-derived CMS has problems in the instability of male sterility under high temperature. The pollen fertility can be restored depending on temperature of the flowering period in the Polima plants. As a result, the F(1) hybrid lack in homogeneities of the typical F(1) hybrid. In some cases where incompatibility between certain nuclear background and Polima CMS has occurred, the resulting F(1) hybrid plants may be small in size and do not express the heterosis which is one of the most important characteristics of the F(1) hybrids.

Other breeders have attempted to introduce Rf genes from radish into rapeseed plants by means of intergeneric crossing. However, these crosses have not been employed practically. Canadian patent application 2,108,230 of Sakai, et al, published on October 12, 1993, claims a fertility restorer gene of a Raphanus plant which is introduced into a Brassica plant by cell fusion or intergeneric cross.

Ogura (OGU) cytoplasmic male sterility is another form of CMS, and used in breeding (Pellan-Delourme et al., 1987, Proc. 7th Int. Rapeseed Conf., Poznan, Poland: 199-203). A fertility restorer for ogura cytoplasmic male sterile plants has been transferred from Raphanus sativum (radish) to Brassica by means of cell fusion or intergeneric cross (US5044066) by Institut National de Recherche Agricole (INRA) in Rennes, France (Pelletier et al., 1987, 7th Int. Rapeseed Conf., Poznau, Poland 113-118). The restorer gene, Rf1 originating from radish, is described in WO 92/05251 and in Delourme et al., 1991, Proc 8th Int. Rapeseed Conf., Saskatoon, Canada. 1506-1510. However, this restorer is inadequate in that restorer inbreds and hybrids carrying this Rf gene have elevated glucosinolate levels and the restorer is closely related to a decrease in seed set - the number of ovules per silique - (Pellan-Delourme et al., 1988, Genome 30:234-238; Delourme et al., 1994, Theor. Appl. Gener. 88: 741-748). In the case of hybrids, the glucosinolate levels are elevated even when the female parent has reduced glucosinolate content.

Canadian patent application 2,143,781 of Yamashita, et al., published on September 11, 1995, claims a hybrid breeding method for crop plants in the family Brassicaceae in which an F1 seed is produced by crossing the female parent of a self-incompatible male sterile line with a male parent. In one embodiment, the male parent possesses a fertility restorer gene. The fertility restorer gene (IM-B) is for MS-N1-derived cytoplasm and was derived from a winter variety (IM line). This was then crossed with a spring double-low line (62We).

A focus of research since the 1970's has been to develop alternative genetic approaches to emasculating plants for the purpose of hybrid seed production. This effort, in part, reflects a desire among farmers to maintain some level of genetic heterogeneity for any given crop. As an example, an inbred line of fertile plants can be converted into a CMS line by crossing it (as male) to a known cytoplasmic male-sterile line and then backcrossing it (as female) to the original inbred line. Given that the CMS-converted line is male-sterile, it must be maintained by crossing to the original inbred line (a "maintainer"). Hybrid seed is produced by growing the CMS-converted inbred line and a second inbred line in isolation, without emasculating. Cytoplasmic male-sterile line can be restored to fertility in a succeeding generation by a nuclear restorer gene. For example, if a CMS-converted line is grown in isolation with a second inbred line carrying a nuclear restorer gene, then the F(1) will be male-fertile and potentially economically valuable.

One approach for maintenance of male sterile plants involves the use of nuclear male-sterile genes and genetic transformation, and specifically uses a cloned nuclear male-sterile gene, such as in Arabidopsis (Aarts, 1993, Nature. 363(6431):715-717) and maize (Albertson et al., 1981, Can. J. Genet. Cytol. 23:195-208). For example, an inbred line of maize, which is homozygous for a mutant allele of a nuclear male-sterile (ms) gene, is genetically engineered to carry a construct comprising an inducible promoter, which, upon induction, allows expression of a wild-type ms gene. The inbred line is maintained in isolation, where it is sprayed with the inducer and allowed to self- and sib-pollinate. Hybrid seed is produced by growing the inbred line with a second inbred line, which carries a wild-type allele of the ms gene, in isolation and in the absence of inducer. Accordingly, the F(1) is heterozygous and, therefore, fertile.

Another approach involves the use of a construct comprising a RNase gene driven by a tapetum-specific promoter (Leemans et al., 1990, Nature 347:737-41). The RNase is active only in the anthers, where it kills the tapetum, i.e., a structure that normally nourishes pollen. Introduction of this construct, via transformation or backcrossing, into an inbred line results in heterozygous dominant male sterility. When the heterozygous dominant male-sterile line is crossed by a N-cytoplasmic line, 50% of the resulting progeny is fertile. Tight linkage of an herbicide resistance gene to the RNAse gene enables elimination of fertile segregants. In practice, the male-sterile line is crossed by a normal progenitor line. The resulting segregating progeny are grown in isolation with a second inbred line. The rows that carry the first inbred line are sprayed with a herbicide. The male-fertile progeny die, leaving only the male-sterile inbred plants from which hybrid seed can be harvested.

International Patent Application WO 90/08830 proposes methods for the production of restorable male-sterile plants in general terms, essentially comprising expression of a) either a gene encoding a protein inhibitor, or b) a so-called killer gene, which said genes are to be expressed in the male flowers, leading to cell death of the anthers and associated tissues. Exemplified killer genes are those which upon expression have an effect on mitochondrial metabolism.

International Patent Application WO 90/08831 discloses that inhibition of cell-respiration by expression of a disrupter gene inhibits mitochondrial function, eventually resulting in the death of the cells in which these genes are expressed. Preferred disrupter proteins are a) the mammalian uncoupling protein (UCP) b) a mutated form of the gene for the β-1 subunit of F&sub1;-ATPase, such that the changes result in the disability of the subunits to assemble into a functional ATP-synthase (c) a mutated, synthetic form of the olil gene encoding subunit 9 of the F&sub0;-ATPase, (d) mutated forms of a mitochondrial transit peptide in order to disrupt protein transport to mitochondria (e) gene-constructs involving a fusion between the β-subunit (ATPase) gene from yeast and the β-galactosidase gene from E.coli, resulting in expression of a disrupting fusion protein. Preferably such expression, according to the specification, should be regulated under the control of a tapetum or pollen-specific promoter.

International Patent Application WO 89/10396, PGS, proposes methods in general terms for obtaining male-sterile plants, by transforming the nuclear genome of the plant with a so-called male-sterility DNA, which is held to comprise DNA which encodes an RNA, or polypeptide capable of disturbing the proper metabolism, functioning, and/or development of any stamen cell in which the male-sterility DNA is expressed, preferably leading thereby to the death of any such stamen cell. Examples of such male-sterility DNA are those encoding DNAses, RNAses, proteases, or enzymes of phytohormone synthesis, such as cytokinin. Alternatively, it is proposed to select male-sterility DNAs from antisense DNAs, 'which encode a strand of DNA complementary to a strand of DNA that is naturally transcribed in the plant's stamen cells'. Apart from the TA29 gene, TA26 gene and the TA13 gene, all tapetum-specific genes derived from tobacco, no clue is given as to what genes are meant. In an article of Koltunow et al., (1990, Plant Cell. 2(12):1201-24), the clones TA13 and TA29 were identified as encoding so-called Glycine Rich Proteins, whereas clone TA26 corresponded with a cDNA of yet unknown nature.

In the European Patent Application EP 0 329 308, Palladin Hybrids, a method to provide male-sterile plants is proposed, comprising producing a genetically transformed female parent, by essentially inserting into the genome of the said plant recombinant DNA sequences comprising antisense DNA which blocks the production of functional pollen grains, or render the developing pollen grains susceptible to a chemical agent or physiological stress which blocks the production of functional pollen grains. Preferably, said antisense genes are expressed under the control of a pollen-specific promoter. Genes which are critical to production of functional pollen grains, according the specification of this Patent Application, are to be selected from genes that are specifically expressed in the microspores, preferably in the premeiotic stage. Examples of microspore specific clones are L4 and L19, derived from Brassica napus. Apart from the general indication to premeiotic genes and the expressly mentioned clones, no further teachings are given with respect to the nature of the genes the expression of which is to be blocked.

The EP 0 335 451 patent, in the name of the 'Vereniging voor Christelijk Wetenschappelijk Onderwijs', describes the inhibition of expression of a chalcone synthase gene in flowers, using an anti-sense gene construct results in altered flower pigmentation. The anti-sense gene in this experiment was placed under the control of the constitutive cauliflower mosaic virus (CaMV) 35S promoter. Plants with altered flower pigmentation were still capable of producing fertile pollen. Chalcone synthase is a key-enzyme in the flavonoid biosynthesis. The enzyme catalyses the stepwise condensation of three acetate residues from malonyl-CoA and one residue of 4-coumaroyl-CoA to yield naringenin chalcone (Heller and Hahlbrock, 1980, Arch. Bioch.Biophys. 200(2):617-19).

Isomerisation and further substitution of this central intermediate ultimately leads to the production of flavonoids. Flavonoids are secondary metabolites that are known to have a key-function in the pigmentation of flowers and fruit. In addition, flavonoids appear to be involved in the defense against phytopathogens (Lamb et al., 1989, Cell 56: 215-224), the protection against UV-light (Schmelzer et al., 1988) and the induction of nodulation (Long et al., 1989). Flavonoids have also been implicated in the regulation of auxin transport (Jacobs and Rubery, 1988) and resistance to insects (Hedin and Waage, 1986, Progress in Clinical & Biological Research. 213:87-100).

This multitude of functions of flavonoids requires a proportionally complex regulation of genes encoding different enzymes of the pathway. The expression of, for example, the anthocyanin biosynthesis genes is flower-specific, light-dependent and developmentally regulated (van Tunen et al., 1988; Koes et al., 1989a). However, expression of these genes in other tissues can be induced by UV-light, wounding or fungal attack (Dixon, 1986; Koes et al., 1989a; Lamb et al., 1989, Cell 56:215-224).

Comparison of the CHS-A promoter with other promoters from flavonoid synthesis genes that are active in immature anther tissue, e.g. CHS-J, DFR-A and CHI-B revealed the presence of a strongly conserved region, designated as the anther box (van Tunen et al., 1989). Deletion analyses of the CHS-A promoter suggested that the anther box itself is not responsible for anther-specific expression, but may be involved in the regulation of anther-specific expression in conjunction with other sequences present in the CHS-A promoter (van der Meer et al., 1990, Plant Mol Biol. 15(1):95-109).

In 1981, Coe et al. 1981 observed that healthy looking white pollen does not function normally in maize, and suggested that pigment synthesis or deposition may be vital to pollen function. However, in the article the involvement of flavonoids in pollen development was not concluded, if not called in doubt. As far as we know, in the years thereafter no conclusive evidence has been reported, regarding a function of flavonoids in the development of viable pollen.

Even though the application of male sterility and fertility restoration has been widely used in hybrid plant production, the use of a restorer gene as a selectable marker for plant transformation remains to be explored. The necessity of a selectable marker in transformation relies on the fact that when new genetic material is to be introduced into a population of cells by transformation, only a certain number of the cells are successfully transformed. It is then necessary to distinguish the genetically transformed cells, so that these cells can be separated from the non-transformed cells of the population.

If an inhibitor or antibiotic or a herbicide compound, is capable of inhibiting the metabolism, growth, or multiplication of the wild-type cell or organism, but not the cell or organism containing a resistant gene, the said selection is generated. In relation to particular enzymes or proteins, "insensitivity" indicates that the enzyme or protein is resistant to specific inhibition by a particular inhibiting compound, for example an antibiotic or herbicide. Selectable marker refers to a nucleotide sequence which, when introduced into the genome of an organism, allows growth of that organism and its progeny under conditions which inhibit growth of the organism lacking the selectable marker. Specific examples of such selectable marker genes are disclosed elsewhere (Weising et al., 1988, Ann. Rev. Genetics 22:421-78). Expression of a selectable marker gene in the transformed cell provides an advantage to the transformed cells over the non-transformed cells in terms of their ability to survive and grow. In the presence of a selection agent, non-transformed cells are subjected to growth inhibition or perhaps even killed, and the transformed cells are able to tolerate and grow. Commonly used selectable markers encode for traits of antibiotic or herbicide resistance. Because the selectable marker gene and the gene of interest are linked, both genes are co-integrated into certain cells in the population. The cells are then cultivated on or in a medium containing the antibiotic or herbicide to which the genetically transformed cells are resistant due to the selectable marker gene. Therefore the transformed cell is selected and may develop into an organ such as a shoot, and further to a seedling. Non-transformed cells-which do not contain the antibiotic or herbicide resistance gene in question-are subjected to growth inhibition or are killed.

The disadvantages of these selectable markers include a) use of antibiotic genes from microorganisms; b) subjecting a great selection pressure to the transformed, and the non-transformed cells during tissue culture, especially in the case of using antibiotic and herbicide resistance markers. The selection bias is towards transformed cells that have a stronger expression of the selectable marker gene during the regeneration phase in tissue culture. In other words, transformed cells that have weak expression of the marker gene are also inhibited or killed during the selection. Weak expression of the marker gene or the nucleotide sequence of interest during tissue culture may not mean low expression of the transgene in the later phase in a plant's life; c) because of the inhibitory effect of the selection agent, e.g. antibiotic or herbicide, on cultured cells, the regeneration process is prolonged.

Accordingly, what is needed in the art are selectable markers that are from plant species and that have less selection bias than what occurs with the use of antibiotic resistance genes or herbicide resistance genes as selectable markers. Also needed are methods of using such fertility restorer nucleic acid selectable markers to identify transformed plants and to select for transgenic plant cells by means of the nucleic acids' capacity to restore pollen production to male sterile plants. Also needed are improved plant lines that can be used as male sterile plants in hybridization systems.

### SUMMARY OF THE INVENTION

This invention fulfills in part the need to identify a new selectable marker for genetic transformation. More specifically, a fertility restoration gene is used as a selectable marker for transformation of a plant that has a male sterile background, provided that the fertility of the plant can be restored by the fertility restorer gene. Moreover, the nucleotide sequence of interest is tightly linked to the fertility restorer nucleic acid, ensuring its co-integration into the target plant genome with the restorer nucleic acid. Expression of the fertility restorer gene in the male sterile plant results in a fertile plant that has normal pollen, and hence viable seeds. Therefore, this new selection relies on the recovery of fertile plant from seeds that contain the restorer nucleic acid and the nucleotide sequence of interest.

This selection strategy is dependent on the fertility restoration with use of the restorer gene. Using a plant with a male sterile background as the transformation target is essential for this invention. Those skilled in the art are well aware of the fact that male sterility and its restoration have long been utilized in plant breeding, specifically for hybrid seed production. A very rich resource of male sterile lines is readily available in many crop species such as corn and Brassica. These materials are selected for their superior agronomic traits in high yields, disease resistance, and possible high grain quality. Transformation of the male sterile lines with any useful trait gene will increase the value of a hybrid plant. However, those male sterile lines may not be readily transformable because of their recalcitrance in tissue culture, and also the lack of a proper selection system.

Several *in planta* transformation strategies may be applicable for transformation of male sterile lines. However, recovery of chimeric transgenic plants from transformed germline cells may be one of the drawbacks. Seeds harvested from Agrobacterium-mediated transformation with a flower-dipping method may overcome the chimeric problem. After germination on medium with a selection agent, those non-transformed seeds are either non-germinable or killed, while transgenic seedlings are recovered. However, this method also requires a selectable marker that is usually an antibiotic or herbicide resistance gene. The presence of such a selectable marker in the final plant product may not be desirable and necessary for many reasons.

Several selectable marker genes originating from plants have been used in plant transformation. One of the examples is the mutated AHAS gene that is resistant to imidazolinone, a herbicide. The mode of selection seems towards the selection of transformed meristematic cells that contribute to the formation of a young plant. However, there is a limitation of this selectable marker as with any other herbicide selectable marker, due to the selection bias applied to the cultured cells. As a consequence, many transformed cells that have low expression of this herbicide resistance gene are probably eliminated, and hence the recovered transgenic events are reduced.

This disclosure describes a new selection strategy that overcomes the disadvantages associated with other selection methods. The transformation target is a male sterile plant that can be cytoplasmic or nuclear male sterile. All of the three cytoplasmic male sterile maize plants, S (USDA), C (Charrua), and T (Texas), can be used in this invention. Two commonly used Brassica cytoplamic male sterile plants, Polima and Ogura, also serve the purpose as a transformation target for this invention. Moreover, a male sterile plant containing a male sterility gene that has been introduced into this plant by genetic transformation, can also be equally used for this purpose. The latter example of male sterile plants have been generated and documented in the literature. For example, expression of an RNase gene driven by a tapetum-specific promotor in a transgenic Brassica plant kills the tapetum, i.e., a structure that normally nourishes pollen and therefore cause male sterility (Leemans et al., 1990, Nature 347: 737-741).

The present disclosure provides that a transformation vector may be designed to contain the nucleotide sequence of interest and a fertility restoration gene within the left and right borders of the T-DNA of a binary vector for Agrobacterium-mediated transformation. A vector for direct DNA delivery methods, such as particle bombardment, can be generated by a tight linkage of the nucleotide sequence of interest with the fertility restorer gene. Transformation may be performed, for example, with Agrobacterium-mediated methods or by particle bombardment, and transgenic plants can be recovered from tissue-cultured cells or tissue. Alternatively, transformation can be targeted at germline cells of a male sterile plant *in planta.* Fertility restoration in a male sterile background can be achieved through the fertility restorer gene, or a gene encoding a mechanism to delete or suppress the male sterility gene. Fertility restoration results in normal pollination and seed production on a plant with the male sterile background. In theory, the only selection criterion is the normal pollen formation and viable seed production from a plant with male sterile background that has been targeted for transformation. Non-transgenic plants will remain male sterile, and hence will not produce seeds. A partially transgenic plant, identified as a chimeric transgenic plant, also produces viable seeds, however only from the flowers that have been derived from the transgenic germline cells. Thereafter, a chimeric transgenic plant produces the viable seeds containing the gene of interest.

Successful application of a fertility restorer gene as a selectable marker for transformation can also be obtained through co-transformation of more than one plasmid vector, with one plasmid having the gene of interest and another plasmid containing the fertility restorer gene. Similarily, selection of transgenic plants with a fertility restorer nucleic acid can also be achieved by using a plasmid vector containing two T-DNAs as known to the skilled in the art.

The present disclosure further provides constructs comprising a male sterility gene flanked by recombinase sites, and methods for using such constructs to produce male sterile lines of plants. The present disclosure further provides constructs to restore fertility to the resultant male sterile plants. These constructs comprise a recombinase gene closely linked to a nucleotide sequence of interest, and flanked by recombinase sites. The target host cells are located in a plant stamen, or more particularly, a plant anther.

The selection strategy as disclosed herein provides several advantages over other transformation selection regimes. First, a plant gene is used as a selectable marker. Therefore, a foreign antibiotic or herbicide resistance marker gene is not needed for the selection, and therefore, is not present in the transgenic plants. Second, if desirable, the gene of interest can be easily segregated from the cytoplasmic male sterile background by crossing the transgenic plant (as a male parent) with a fertile female parent after recovery of the transgenic seeds. Third, any trait associated with the gene of interest, such as stress tolerance, disease resistance, and yield, can be incorporated into the hybrid seed production, since the male sterility restoration gene is linked with the gene of interest. A transgenic male fertile line containing the gene of interest and the restorer gene can be used as a male parent with a male sterile female line to produce hybrid seeds. Fourth, because there is no chemical selection pressure applied during transgenic plant recovery, the selected transgenic events may demonstrate a more random selection. Chemical selection may cause elimination of many integration events that are in favor of the selection pressure during the chemical selection. Fifth, the use of male fertility restoration as a selection is more practical for germline/*in planta* transformation procedures, because only a visual phenotype (seed production) is used as a selection criterion.

Lastly, a restorer gene per se is not necessary for this invention with application of a recombinase. A transgenic male sterile plant can be generated by transforming a plant with a "killer" gene, i.e. a gene that causes abortion of pollen development. This "killer" gene is a chimeric DNA molecule that includes a male sterile gene flanked by specific recombinase sites outside the male sterile gene nucleotide sequence. A second transformation can be performed on this resultant male sterile plant by introduction of a vector containing a recombinase gene and the nucleotide sequence of interest. The recombinase removes the male sterility gene, and replaces it with the nucleotide sequence of interest. The consequence is that the fertility is restored (selection), and the nucleotide sequence of interest (transgene) is incorporated into the plant genome. This strategy can be applicable for the targeted transformation once a series of male sterile tagged lines are generated. The final transgenic plants do not contain any selectable marker.

The present disclosure also provides plant cells, plant parts, plant seeds and plants comprising the nuclear fertility restorer genes, proteins and vectors described herein. A plant seed comprises a nuclear fertility restorer nucleic acid, and accordingly, the plant seed is true breeding for the ability to restore fertility in a male-sterile plant. The disclosure further provides an agricultural product produced by any of the below-described plants, plant parts, or plant seeds.

### DETAILED DESCRIPTION OF THE INVENTION

The present invention may be understood more readily by reference to the following detailed description of the preferred embodiments of the invention and the Examples included herein.

This invention fulfills in part the need to identify new selectable markers that overcome the disadvantages of the selectable markers known in the art. This invention also fulfills in part the need to provide improved sterility and restorer lines for plants, and canola in particular. The present disclosure further provides constructs comprising the new selectable markers for use in creating the improved sterility and restorer lines.

The fertility restorer nucleic acid molecules presented herein that function as restorer genes can be used as selection markers to identify transformed plant cells. For example, a sequence that functions as a restorer gene could be combined with another nucleotide sequence of interest in a transformation vector. The vector could then be introduced into plant cells by any of a number of methods, such as Agrobacterium-mediated transformation. If the plant cells into which the DNA is introduced are male sterile, the anthers that form on the regenerated plants will normally not produce any pollen, and no seed will form from these flowers by self-pollination. If, however, the anthers form from cells that have acquired a restorer gene and the associated nucleotide sequence of interest, the anthers will produce pollen and these flowers will form seeds. This provides a powerful selection system for the identification of transformed plants or parts of plants. The use of restorer genes, which are purely normal plant genes, as selection markers may have some advantages over other currently used selection markers with respect to regulatory issues. The use of restorer genes as selection markers is not intended to be limited to radish, petunia, or maize, but rather could apply to any restorer gene for any system in any plant species.

As used herein, the term "fertility restorer nucleic acid" refers to a nucleic that when expressed in a male sterile plant can restore male fertility to the plant. The disclosure provides an isolated plant transformation vector comprising a fertility restorer nucleic acid as described below, wherein expression of the vector in a host plant results in the plant's increased production of viable pollen. Said host cells are located in a plant stamen, or more particularly, a plant anther.

The disclosure provides transformation vectors comprising a fertility restorer nucleic acid and à nucleotide sequence of interest. In a preferred embodiment, the fertility restorer nucleic acid is selected from the group consisting of a radish fertility restorer nucleic acid, a petunia fertility restorer nucleic acid, and a maize fertility restorer nucleic acid. Said fertility restorer nucleic acid and the nucleotide sequence of interest are each under the control of separate heterologous promoters. As used herein, a "heterologous promoter" refers to a nucleotide sequence that is not naturally associated with the nucleic acid to which it is attached and that is capable of promoting expression of a gene or antisense gene, or nucleotide sequences thereof, said expression being in the form of an RNA molecule and/or polypeptide. In one embodiment, the heterologous promoter is selected from the group consisting of a constitutive promoter, an inducible promoter, a gamete-specific promoter, a tissue-preferred promoter, and an organ-preferred promoter

The present disclosure also provides transformation vectors comprising a male sterility nucleic acid under the control of a heterologous promoter and flanked at both ends by recombinase sites. As used herein, a "male sterility nucleic acid" is nucleic acid whose expression in a plant causes nuclear or cytoplasmic sterility in the plant. Conversely, by removing or inactivating expression of a "male sterility nucleic acid," male fertility is restored in the plant. The present disclosure further provides a transformation vector comprising a nucleic acid encoding a recombinase and a nucleotide sequence of interest, flanked at both ends by recombinase sites. A male sterile plant can be generated by transforming a male fertile plant with the previous transformation construct comprising the male sterility nucleic acid. Expression of the recombinase of the latter vector in such a male sterile plant can restore the plant's male fertility Accordingly, the restoration of a male sterile plant's male fertility due to the expression of the recombinase can be used as a selection marker to identify transformed plant cells comprising the nucleotide sequence of interest.

As used herein, a "nucleotide sequence of interest" refers to a nucleotide sequence encoding a polypeptide for some desired trait. For example, the nucleotide sequence of interest is an altered Als nucleic acid that confers increased tolerance to imidazolinone herbicide to the plant in which it is expressed. Said nucleotide sequence may encode a protein that confers increased stress tolerance to the plant in which it is expressed.

As used herein, the term "recombinase" refers to an enzyme that catalyzes recombination between two or more recombination sites. Recombinases useful in the present invention catalyze recombination at specific recombination sites which are specific polynucleotide sequences that are recognized by a particular recombinase. A number of different site specific recombination systems can be used with the present invention. These include, but are not limited to, Cre, FLP, φC31, R, Gin, Pin, and β-recombinase. Said nucleotide sequences encoding these recombinases are shown in the Appendix. Site-specific recombinases, such as Flp and Cre of the integrase family and φC31 of the recombinase family, recognize specific DNA sequences and perform specific recombinations of the nucleic acid sequence by restricting and ligating the nucleic acid at these sites. The placement and orientation of these target sites determines the type of recombination that occurs. In particular, directly oriented target sites facilitate excision of intervening DNA in the presence of recombinase whereas inverted target sites facilitate inversion of the intervening DNA (Kilby et al. 1995 Plant Journal 8:637-652; Kilby et al. 1993 Trends in Genetics 9:413-421; Pósfai et al. 1994 Nucleic Acids Research 22:2392-2398; Stark et al. 1992 Trends in Genetics 8:432-439).

Site-specific recombinase genes, including Flp, Cre and φC31, are used as tools for genetic engineering because of their simplicity and precision. Flp and Cre recombinase have been successfully employed in a broad range of organisms, including plants (Reviewed by Kilby et al. 1993 Trends in Genetics 9:413-421). The Flp and Cre recombinases recognize distinct 34 bp sites, called FRT and loxP, respectively; the large size and structure of these sites make random occurrences in higher eukaryotic genomes unlikely (Dymecki, 1996, PNAS 93:6191-6196, Senacoff et al., 1988, Journal of Molecular Biology 201:405-421), but nonetheless, their use for mutagenesis has been proposed. Another useful irreversible recombination system described in the prior art is the *Streptomyces* phage φC31 recombination system. A 68 kDa integrase protein recombines an *attB* site with an *attP* site. These sites share only three base pairs of homology at the point of cross-over. This homology is flanked by inverted repeats, presumably binding sites for the integrase protein. The minimal known functional size for both the φC31 *attB* and *attP* is approximately 30 to 40 base pairs. Unlike other recombinase systems, the φC31 integration reaction is simple in that it does not require a host factor.

The Cre-*lox* system of bacteriophage P1, and the FLP-FRT system of *Saccharomyces cerevisiae* have been widely used for transgene and chromosome engineering in animals and plants (reviewed by Sauer (1994) Curr. Opin. Biotechnol. 5: 521-527; Ow (1996) Curr. Opin. Biotechnol. 7: 181-186). Other systems that operate in animal or plant cells include the following: 1) the R-RS system from *Zygosaccharomyces rouxii* (Onouchi et al. (1995) Mol. Gen. Genet. 247: 653-660), 2) the Gin-*gix* system from bacteriophage Mu (Maeser & Kahmann (1991) Mol. Gen. Genet. 230: 170-176) and, 3) the β recombinase-six system from bacterial plasmid pSM19035 (Diaz et al. (1999) J. Biol. Chem. 274: 6634-6640).

Recombinases have been utilized for a diverse set of applications in transgenic plants including control of transgene expression by the excision of blocker DNA by a recombinase resulting in the activation of gene expression (U.S. Patent No. 5,723,765). Recombinases have also been used to stimulate site-specific integration, mutation, inversion and deletion (Dunaway et al., 1997, Molecular & Cellular Biology 17:182-189; Dymecki, 1996, PNAS 93:6191-6196; Kilby et al., 1995, Plant Journal 8:637-652; Kilby et al., 1993, Trends in Genetics 9:413-421, Ow and Medberry, 1995, Crit. Rev. Plant Sci. 14:239-261; Pósfai et al., 1994, Nucleic Acids Research 22:2392-2398; Senacoff et al., 1988, Journal of Molecular Biology 201:405-421; Stark et al., 1992, Trends in Genetics 8:432-439, Walters et al., 1999, Molecular & Cellular Biology 19:3714-3726).

The nuclear fertility restorer genus is derived from a radish, comprises pentatricopeptide (PPR) motifs, and is able to restore fertility in a male-sterile plant. Alternatively, the nuclear fertility restorer genus is lacking genes associated with increased glucosinolate traits , the male-sterile plant comprises the *ogu* male sterility determinant and the nuclear fertility restorer gene is derived from *Raphanus sativum*, the fertility restorer gene is from petunia or maize.

The compositions of, and methods of constructing, nucleic acid molecules for successful transformation of plants are well known to those skilled in the art. For example, the uses of suitable nucleic acid components such as promoters, polyadenylation sequences, selectable marker sequences, reporter sequences, enhancers, introns, and the like, as well as references providing the specific compositions of those components are described elsewhere (Weising et al., Ann. Rev. Genetics 22:421-478 (1988)). In addition, suitable methods of construction are described elsewhere (Sambrook J., et al., Molecular Cloning: A Laboratory Manual, 2nd ed., Cold Spring Harbor Laboratory Press (1989)). Since the specific composition of the promoter, terminator, reporter gene and introns is not central to the present invention, it is important to note that the same or similar compositions and methods can be used herein to produce nucleic acid molecules that are useful for transforming any plant.

It is to be understood that the nucleic acid molecules can be delivered into plant cells by several techniques, for example, with particle bombardment and Agrobacterium tumefaciens-mediated transformation. These two methods are well known to those skilled in the art. Suitable particle bombardment instruments and particle bombardment methods are provided elsewhere (Sanford et al., 1987; Heiser W., "Optimization of Biolistic.RTM. transformation using the helium-driven PDS-1000/He system" in US/EG Bulletin 1688, BIO-RAD; and Dunder et al., "Comparison of performance characteristics of different Biolistic.RTM. devices" in US/EG Bulletin 1689, BIO-RAD). A protocol for the use of a high pressure helium tank instrument (Biolistic.RTM. PDS-1000/He Particle Delivery System) is provided by the manufacture (BIO-RAD, Hercules Calif.). Briefly, the particle bombardment process, also referred to as a biolistic process, delivers a desired nucleic acid molecule to a cell by using very small particles, such as tungsten and gold particles that have been coated with the nucleic acid molecule. When the particles are coated with the nucleic acid molecule and accelerated to a suitable velocity, one or more of the particles penetrate into the plant target cells, and release the nucleic acid from the particle within the cell. Some of the recipient cells that survive the damage caused by the bombardment, retain the introduced nucleic acid molecule, integrate the introduced nucleic acid molecule into its genome, and express the nucleic acid molecule.

Agrobacterium-mediated transformation can also be used for delivery of a nucleic acid molecule to plant cells. Brassica plants are transformed using *Agrobacterium tumefaciens* or *Agrobacterium rhizogenes,* preferably *Agrobacterium tumefaciens.* Agrobacterium-mediated transformation utilizes the natural ability of *A. tumefaciens* and *A. rhizogenes* to transfer a DNA molecule into plant chromosomes. Agrobacterium is a plant pathogen that transfers a set of genes encoded in a region called T-DNA of the Ti and Ri plasmids of *A. tumefaciens* and *A. rhizogenes,* respectively, into plant cells. The typical result of transfer of the T-DNA from a Ti plasmid is a crown gall formation in the infected plant in which the T-DNA is stably integrated into a host chromosome. Integration of the T-DNA of a Ri plasmid into the host chromosomal DNA results in hairy root disease in the host plant. The genes in the T-DNA region that causes disease in the host plant can be deleted or disarmed without loss of the ability of T-DNA transfer and integration. The two border sequences of the T-DNA are important for its transfer and integration into the host plant genome.

Gene transfer by means of engineered Agrobacterium strains has become a routine task to those skilled in the art for many dicotyledonous crop plants, and monocotyledonous plants. The nucleic acid to be transferred is incorporated into the T-region and is flanked by T-DNA border sequences. A variety of Agrobacterium strains are known in the art particularly for dicotyledon transformation. Such Agrobacterium can be used in the methods of the invention (Hooykaas, 1989; Smith et al., 1995; Chilton, 1993; Mollony et al., 1993; Ishida et al., 1996; and Komari et al., 1996).

The Agrobacterium-mediated transformation process may comprise several steps including infection, co-cultivation, selection, and regeneration. An optional pre-culture step may be included prior to the infection step. The pre-culture step involves culturing the target tissues prior to the infection step on a suitable medium. The pre-culture period may vary from about 1 to 5 days.

In the infection step, the cells to be transformed are exposed to Agrobacterium by contacting with the Agrobacterium, typically in a liquid medium. The concentration of Agrobacterium used in the infection step and co-cultivation step can affect the transformation frequency. Likewise, high concentrations of Agrobacterium may cause damage to the tissue to be transformed and result in a reduced transformation frequency. Thus, the concentration of Agrobacterium may vary depending on the Agrobacterium strain utilized, the tissue being transformed, the Brassica species being transformed, and the like. To optimize the transformation protocol for a particular Brassica species or tissue, the tissue to be transformed can be incubated with various concentrations of Agrobacterium.

The tissue to be transformed is generally added to the Agrobacterium suspension in a liquid contact phase containing a concentration of Agrobacterium to optimize transformation efficiencies. Infection is generally allowed to proceed for a period of time from 1 to 30 minutes prior to the co-cultivation step. The infection conditions can be optimized to achieve the highest level of infection and transformation by Agrobacterium. For example, the plant cells are subjected to osmotic pressure (e.g., 0.6 M mannitol) during the infection and co-cultivation steps. Additionally, to enhance transformation efficiency, tissue may be cultured in medium containing an auxin and an cytokinin, such as IAA and BAP, to promote cell proliferation because it is believed that Agrobacterium integrates into the genome during mitosis. As further alternatives, tissue wounding, vacuum pressure, or cultivation in medium containing acetosyringone can be employed to promote the transformation efficiency.

In the co-cultivation step, the cells to be transformed are co-cultivated with Agrobacterium, typically on a solid medium. Any suitable medium, such as MS medium containing 3% sucrose and 0.8% agar, may be utilized. The optimal co-cultivation time varies with the particular tissue, and 1 to 3 days are preferable for meristem tissue in Brassica. Co-cultivation may be carried out in the dark or under low light conditions to enhance the transformation efficiency. Additionally, as described above for the inoculation step, co-cultivation can be done on medium containing IAA or cytokinins or acetosyringone to promote transformation efficiency. Following the co-cultivation step, transgenic shoots may be selected and regenerated into plantlets, as known for those skilled in the art, on medium containing selection agent and antibiotics for eliminating Agrobacterium.

In order to avoid problems associated with tissue culture-based regeneration of transgenic plants from transformed cells, *in planta* transformation methods have been developed (Bechtold et al., 1993; Chang et al., 1994, Plant J. 5:551-558; Clough and Bent, 1998, Plant J. 16:735-743; Feldmann and Marks, 1987, Mol. Gen. Genet. 208:1-9; Katavic et al., 1994, Mol. Gen. Genet. 245:363-370; Richardson et al., 1998, Aust. J. Plant Physiol. 25:125-130; Trieu et al., 2000, Plant J. 22:531-541). The development and application of these transformation procedures contribute to a major advance in overall plant transformation capability.

Briefly, Agrobacterium suspension cells are infiltrated into seedlings or flowers of the seedlings with the assistance of a vacuum force (Bechtold et al., 1993; Trieu et al., 2000, Plant J. 22:531-541). The plant developmental stages are critical for the success of this technique. For example, flowers of *Medicago truncatula* at a stage when the plants have flower buds and a few open flowers, are submerged in a suspension of Agrobacterium, and the bacteria are vacuum-infiltrated into the plant (Trieu et al., 2000, Plant J. 22:531-541). Following infiltration, plants are able to survive and produce some seeds. Subsequent screening of the seeds with a selection agent, e.g. antibiotic or herbicide, or molecular biological analysis is required. The screening of T1 seeds with kanamycin seems not to be effective in *M. truncatula* (Trieu et al., 2000, Plant J. 22:531-541).

The present disclosure also provides plant cells, plant parts, plant seeds and plants comprising the fertility restorer nucleic acids, proteins, and vectors described herein. Said plant seed comprises a nuclear fertility restorer nucleic acid, and accordingly, the plant seed is true breeding for the ability to restore fertility in a male-sterile plant. The disclosure further provides an agricultural product produced by any of the below-described plants, plant parts or plant seeds.

The disclosure additionally provides a method of producing a hybrid plant comprising crossing a male-sterile plant with a restorer plant, wherein the restorer plant contains a nuclear fertility restorer nucleic acid described herein. The present disclosure also provides a method of restoring male fertility in a plant comprising introducing a nuclear fertility restorer nucleic acid into a male-sterile plant. The present disclosure also provides a method of increasing the production of viable pollen in a plant, including introducing a nuclear fertility restorer nucleic acid into a plant. The present disclosure also provides methods of using genetic markers from the sequences described herein to determine the presence of a nuclear fertility restorer genus in a plant.

As used herein, the term "male-sterile plant" refers to a plant wherein the function of the male organs is disrupted or blocked, or more particularly, wherein the male organs are unable to produce viable pollen. The term "male-sterile" includes genic or nuclear male sterility and cytoplasmic-genetic male sterility. The terms "cytoplasmic-genetic male sterile" and "cytoplasmic male sterile" are used interchangeably herein and refer to male sterility resulting from the dysfunctioning of one or more cytoplasmic genes (e.g. mitochondria genes). In one embodiment, the nucleic acids and proteins are used to restore fertility in a cytoplasmic male-sterile, or CMS, plant. Said cytoplasmic male-sterile plant comprises a cytoplasmic male sterility determinant selected from the group consisting of *pol, nap,* Toumefortii, Kosena and ogu. In yet another embodiment, the cytoplasmic male sterility determinant is *ogu.* As used herein, the terms "genic male sterility," "chromosomal male sterility," and "nuclear male sterility," are used interchangeably and refer to sterility resulting from the presence of two recessive alleles of a nuclear male sterility locus in a plant.

The male-sterile plants can be selected from maize, wheat, rye, oat, triticale, rice, barley, soybean, peanut, rapeseed, canola, pepper, sunflower, Vicia species, pea, alfalfa, bushy plants (coffee, cacao, tea), Salix species, radish, sorghum, pearl millet, cotton, and tobacco. It is preferable however that the male-sterile plant is a canola plant selected from the group of Brassica species consisting of *Brassica napus, Brassica rapa (*or *campestris), Brassica oleracea, Brassica nigra, Brassica juncea, Sinapis alba,* and *Brassica carinata.* In a more preferred embodiment, the male-sterile plant is *Brassica napus.*

The present disclosure encompasses vectors comprising an *Rfo* gene derived from a species of radish, including but not limited to, *Raphanus sativum. Said Rfo* gene is derived from *Raphanus sativum.* Accordingly, the disclosure provides an *Rfo* gene that, upon its introduction into a male-sterile plant, is able to increase the plant's production of pollen and/or restore the fertility of the plant. The disclosure encompasses an *Rfo* gene derived from a species of radish, including but not limited to, *Raphanus sativum.*

Alternatively, the fertility restorer nucleic acid is derived from *Petunia.* Accordingly, the disclosure provides a *Petunia* fertility restorer nucleic acid that, upon its introduction into a male-sterile plant, is able to increase the plant's production of pollen and/or restore the fertility of the plant.

Alternatively, the fertility restorer nucleic acid is derived from maize. Accordingly, the disclosure provides an maize fertility restorer nucleic acid that, upon its introduction into a male-sterile plant, is able to increase the plant's production of pollen and/or restore the fertility of the plant.

As used herein, the terms "gene," "nucleic acid," and "nucleic acid molecule" are used interchangeably herein and are intended to include DNA molecules (e.g., cDNA or genomic DNA) and RNA molecules (e.g., mRNA) and analogs of the DNA or RNA generated using nucleotide analogs. This term also encompasses untranslated sequence located at both the 3' and 5' ends of the coding region of the gene: up to at least about 1000 nucleotides of sequence upstream from the 5' end of the coding region and up to at least about 200 nucleotides of sequence downstream from the 3' end of the coding region of the gene. The nucleic acid molecule can be single-stranded or double-stranded, but preferably is double-stranded DNA.

An "isolated" nucleic acid molecule is one that is substantially separated from other nucleic acid molecules that are present in the natural source of the nucleic acid (i.e., sequences encoding other proteins). Preferably, an "isolated" nucleic acid is free of some of the sequences that naturally flank the nucleic acid (i.e., sequences located at the 5' and 3' ends of the nucleic acid) in its naturally occurring replicon. For example, a cloned nucleic acid is considered isolated. An *Rfo* nucleic acid is isolated when it is separated from all or part of the glucosinolate gene, for example in *Raphanus sativum*. Said isolated *Rfo* nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived (e.g., a *Raphanus sativum* cell). A nucleic acid is also considered isolated if it has been altered by human intervention, or placed in a locus or location that is not its natural site, or if it is introduced into a cell by Agrobacterium-mediated transformation. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

Specifically excluded from the definition of "isolated nucleic acids" are: naturally-occurring chromosomes (such as chromosome spreads), artificial chromosome libraries, genomic libraries, and cDNA libraries that exist either as an in vitro nucleic acid preparations or as a transfected/transformed host cell preparation, wherein the host cells are either an *in vitro* heterogeneous preparation or plated as a heterogeneous population of single colonies. Also specifically excluded are the above libraries wherein a specified nucleic acid makes up less than 5% of the number of nucleic acid inserts in the vector molecules. Further specifically excluded are whole cell genomic DNA or whole cell RNA preparations (including whole cell preparations that are mechanically sheared or enzymatically digested). Even further specifically excluded are the whole cell preparations found as either an *in vitro* preparation or as a heterogeneous mixture separated by electrophoresis wherein the nucleic acid of the invention has not further been separated from the heterologous nucleic acids in the electrophoresis medium (e.g., further separating by excising a single band from a heterogeneous band population in an agarose gel or nylon blot).

A nucleic acid molecule as disclosed herein, or a portion thereof, can be isolated using standard molecular biology techniques and the sequence information provided herein. For example, a *Rfo* cDNA can be isolated from a *Raphanus sativum* library using all or a portion of the sequence herein. Moreover, a nucleic acid molecule encompassing all or a portion of sequence herein can be isolated by the polymerase chain reaction using oligonucleotide primers designed based upon this sequence. For example, mRNA can be isolated from radish cells (e.g., by the guanidinium-thiocyanate extraction procedure of Chirgwin et al., 1979 Biochemistry 18:5294-5299) and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon the nucleotide sequence shown herein. A nucleic acid molecule as disclosed herein can be amplified using cDNA or, alternatively, genomic DNA, as a template and appropriate oligonucleotide primers according to standard PCR amplification techniques. The nucleic acid molecule so amplified can be cloned into an appropriate vector and characterized by DNA sequence analysis. Furthermore, oligonucleotides corresponding to a *Rfo* nucleotide sequence can be prepared by standard synthetic techniques, e.g., using an automated DNA synthesizer.

An isolated nucleic acid molecule as disclosed herein comprises one of the nucleotide sequences described herein. It is to be understood that sequences described herein comprise whole genomic fragments isolated from genomic DNA. Accordingly, the nucleic acid molecules as disclosed herein may contain both coding regions and 5' and 3' untranslated regions that can include promoters and other regulatory sequences. Alternatively, the nucleic acid molecules as disclosed herein can comprise only the coding region. A coding region of these sequences is indicated as an "ORF position". The present disclosure also includes coding nucleic acids that encode proteins as described herein.

Moreover, the nucleic acid molecule as disclosed herein can comprise only a portion of the coding region of the sequences shown herein, for example, a fragment which can be used as a probe or primer or a fragment encoding a biologically active portion of the protein.

Portions of proteins encoded by the nucleic acid molecules of the invention are preferably biologically active portions of one of the proteins described herein. As used herein, the term "biologically active portion of" a protein is intended to include a portion, e.g., a domain/motif, of the protein that participates in the restoration of fertility in a cytoplasmic male-sterile plant. In a preferred embodiment, the biologically active portion of a Rfo protein comprises one or more PPR motifs. To determine whether a protein, or a biologically active portion thereof, can restore fertility in a male-sterile plant, a fertility analysis of a plant comprising the protein may be performed. Such analysis methods are well known to those skilled in the art. More specifically, nucleic acid fragments encoding biologically active portions of a fertility restorer protein can be prepared by isolating a portion of sequences shown herein, introducing the isolated portion of nucleic acid into a male-sterile plant and assessing whether male-fertility is restored. A determination as to whether male-fertility is restored in a plant can be made, for example, by 1) visually assessing an increase in the production of pollen as compared to a male-sterile plant or 2) determining that the plant can self-fertilize as evidenced by placing a bag over a flower on the plant and finding an increase of seed therein as compared to a male-sterile plant. It is to be understood that a male-sterile plant containing an *ogu* cytoplasmic male sterility determinant can produce a small amount of pollen. For example, restoration of fertility in a male-sterile plant is indicated by an increase in the plant's pollen production by at least 10%.

Biologically active portions of a fertility restorer protein are encompassed by the present invention and include peptides comprising amino acid sequences derived from the amino acid sequence of a fertility restorer protein, or the amino acid sequence of a protein homologous to a fertility restorer protein, which includes fewer amino acids than a full length fertility restorer protein or the full length protein which is homologous to a fertility restorer protein, and exhibit at least one activity of a fertility restorer protein. Typically, biologically active portions (e.g., peptides which are, for example, 5, 10, 15, 20, 30, 35, 36, 37, 38, 39, 40, 50, 100 or more amino acids in length) comprise a domain or motif with at least one activity of a fertility restorer protein. Moreover, other biologically active portions in which other regions of the protein are deleted, can be prepared by recombinant techniques and evaluated for one or more of the activities described herein. Preferably, the biologically active portions of a fertility restorer protein include one or more PPR domains/motifs or portions thereof and are able to restore fertility in a male-sterile plant

The disclosure also provides fertility restorer chimeric or fusion proteins. As used herein, a fertility restorer "chimeric protein" or "fusion protein" comprises a fertility restorer polypeptide operatively linked to a non- fertility restorer polypeptide. A fertility restorer polypeptide refers to a polypeptide having an amino acid sequence corresponding to a fertility restorer protein, whereas a non- fertility restorer polypeptide refers to a polypeptide having an amino acid sequence corresponding to a protein which is not substantially homologous to the fertility restorer, e.g., a protein that is different from the fertility restorer and is derived from the same or a different organism. As used herein with respect to the fusion protein, the term "operatively linked" is intended to indicate that the fertility restorer polypeptide and the non- fertility restorer polypeptide are fused to each other so that both sequences fulfill the proposed function attributed to the sequence used. The non-fertility restorer polypeptide can be fused to the N-terminus or C-terminus of the fertility restorer polypeptide. For example, the fusion protein is a fertility restorer protein containing a heterologous signal sequence at its N-terminus.

Preferably, a fertility restorer chimeric or fusion protein is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining and enzymatic ligation. For example, the fusion gene can be synthesized by conventional techniques including automated DNA synthesizers. Alternatively, PCR amplification of gene fragments can be carried out using anchor primers that give rise to complementary overhangs between two consecutive gene fragments which can subsequently be annealed and re-amplified to generate a chimeric gene sequence (see, for example, Current Protocols in Molecular Biology, Eds. Ausubel et al. John Wiley & Sons: 1992). Moreover, many expression vectors are commercially available that already encode a fusion moiety (e.g., a GST polypeptide). A fertility restorer encoding nucleic acid can be cloned into such an expression vector such that the fusion moiety is linked in-frame to the fertility restorer protein.

In addition to fragments and fusion proteins of the fertility restorer proteins described herein, the present disclosure includes homologs and analogs of naturally occurring fertility restorer proteins and fertility restorer encoding nucleic acids in a plant. "Homologs" are defined herein as two nucleic acids or proteins that have similar, or "homologous", nucleotide or amino acid sequences, respectively. Homologs include allelic variants, orthologs, paralogs, agonists and antagonists of fertility restorer proteins as defined hereafter. The term "homolog" further encompasses nucleic acid molecules that differ from the nucleotide sequence shown herein (and portions thereof) due to degeneracy of the genetic code and thus encode the same fertility restorer protein as that encoded by the nucleotide sequences shown herein. As used herein a "naturally occurring" fertility restorer protein refers to a fertility restorer amino acid sequence that occurs in nature.

Nucleic acid molecules corresponding to natural homologs such as allelic variants, orthologs and paralogs and natural analogs of a fertility restorer cDNA can be isolated based on their identity to the fertility restorer nucleic acids described herein. These natural homologs and analogs can be isolated using fertility restorer cDNAs, or a portion thereof, as a hybridization probe according to standard hybridization techniques under stringent hybridization conditions. In an alternative embodiment, homologs of the fertility restorer protein can be identified by screening combinatorial libraries of mutants, e.g., truncation mutants, of the fertility restorer nucleic acids for fertility restorer protein agonist or antagonist activity. For example, a variegated library of fertility restorer variants is generated by combinatorial mutagenesis at the nucleic acid level and is encoded by a variegated gene library. A variegated library of fertility restorer variants can be produced by, for example, enzymatically ligating a mixture of synthetic oligonucleotides into gene sequences such that a degenerate set of potential fertility restorer sequences is expressible as individual polypeptides, or alternatively, as a set of larger fusion proteins (e.g., for phage display) containing the set of fertility restorer sequences therein. There are a variety of methods that can be used to produce libraries of potential fertility restorer homologs from a degenerate oligonucleotide sequence. Chemical synthesis of a degenerate gene sequence can be performed in an automatic DNA synthesizer, and the synthetic gene is then ligated into an appropriate expression vector. Use of a degenerate set of genes allows for the provision, in one mixture, of all of the sequences encoding the desired set of potential fertility restorer sequences. Methods for synthesizing degenerate oligonucleotides are known in the art (see, e.g., Narang, S.A., 1983 Tetrahedron 39:3; Itakura et al., 1984 Annu. Rev. Biochem. 53:323; Itakura et al., 1984 Science 198:1056; Ike et al., 1983 Nucleic Acid Res. 11:477).

In addition, libraries of fragments of the fertility restorer coding regions can be used to generate a variegated population of fertility restorer fragments for screening and subsequent selection of homologs of a fertility restorer. For example, a library of coding sequence fragments can be generated by treating a double stranded PCR fragment of a fertility restorer coding sequence with a nuclease under conditions wherein nicking occurs only about once per molecule, denaturing the double stranded DNA, renaturing the DNA to form double stranded DNA, which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the fertility restorer proteins.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of fertility restorer homologs. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify fertility restorer homologs (Arkin and Yourvan, 1992 PNAS 89:7811-7815; Delgrave et al., 1993 Protein Engineering 6(3):327-331). In another embodiment, cell based assays can be exploited to analyze a variegated fertility restorer library, using methods well known in the art.

Preferably, the above described fertility restorer homologs retain the same biological activity as the fertility restorer proteins shown herein, and more preferably, the fertility restorer homologs restore fertility in a male-sterile plant. To determine the percent homology of two amino acid sequences, the sequences are aligned for optimal comparison purposes (e.g., gaps can be introduced in the sequence of one protein for optimal alignment with the other protein). The amino acid residues at corresponding amino acid positions are then compared. When a position in one sequence is occupied by the same amino acid residue as the corresponding position in the other sequence, then the molecules are homologous at that position (i.e., as used herein amino acid or nucleic acid "homology" is equivalent to amino acid or nucleic acid "identity"). The same type of comparison can be made between two nucleic acid sequences.

The percent homology between the two sequences is a function of the number of identical positions shared by the sequences (i.e., % homology = numbers of identical positions/total numbers of positions x 100). Preferably, the isolated fertility restorer protein homologs included in the present invention are at least about 50-60%, preferably at least about 60-70%, and more preferably at least about 70-80%, 80-90%, 90-95%, and most preferably at least about 96%, 97%, 98%, 99% or more homologous to an entire amino acid sequence shown in herein. Alternatively, the isolated fertility restorer protein homologs included in the present invention are at least about 50-60%, preferably at least about 60-70%, and more preferably at least about 70-80%, 80-90%, 90-95%, and most preferably at least about 96%, 97%, 98%, 99% or more homologous to an entire amino acid sequence encoded by a nucleic acid sequence shown herein. Alternatively, the isolated fertility restorer protein homologs have homology over at least 15 contiguous amino acid residues, more preferably at least 25 contiguous amino acid residues, and most preferably at least 35 contiguous amino acid residues of the sequences shown herein. In a further preferred embodiment, the Fertility restorer homologs have greater than 90% homology over the PPR motif.

Alternatively, an isolated fertility restorer nucleic acid homolog of the invention comprises a nucleotide sequence which is at least about 50-60%, preferably at least about 60-70%, more preferably at least about 70-80%, 80-90%, or 90-95%, and even more preferably at least about 95%, 96%, 97%, 98%, 99% or more homologous to a nucleotide sequence shown herein, or a portion thereof. The preferable length of sequence comparison for nucleic acids is at least 75 nucleotides, more preferably at least 100 nucleotides and most preferably the entire coding region of the nucleic acid.

The determination of the percent homology between two sequences is accomplished using a mathematical algorithm. Preferably, the percent homology between two sequences is determined using the mathematical algorithm of Karlin and Altschul (1990 Proc. Natl. Acad. Sci. USA 90:5873-5877). Such an algorithm is incorporated into the NBLAST and XBLAST programs of Altschul, et al. (1990 J. Mol. Biol. 215:403-410). Accordingly, the present disclosure includes a Fertility restorer nucleic acid homolog having at least 50% homology with the nucleotide sequence shown herein as determined using the NBLAST program, score=100, wordlength=12. Additionally, the present invention includes a Fertility restorer amino acid homolog having at least 70% homology with the amino acid sequence shown herein as determined using the XBLAST program, score=50, wordlength=3. When BLAST programs are used to determine percent homology, Gapped BLAST is utilized as described in Altschul et al. (1997 Nucleic Acids Res. 25:3389-3402). When utilizing BLAST and Gapped BLAST programs, the default parameters of the respective programs (e.g., XBLAST and NBLAST) are used.

Alternatively, the percent homology between two sequences is determined using the mathematical algorithm of Smith and Waterman. Alternatively, the percent homology between two sequences is determined using the mathematical algorithm of Myers and Miller (CABIOS 1989). The Myers and Miller algorithm is incorporated into the ALIGN program (version 2.0) that is part of the GCG sequence alignment software package. When utilizing the ALIGN program for comparing amino acid sequences, a PAM120 weight residue table, a gap length penalty of 12 and a gap penalty of 4 is used to obtain fertility restorer amino acid homologs.

Finally, homology between nucleic acid sequences can be determined using hybridization techniques known to those of skill in the art. Accordingly, an isolated fertility restorer nucleic acid molecule comprises a nucleotide sequence which hybridizes, e.g., hybridizes under stringent conditions, to the nucleotide sequence shown herein or a portion thereof. More particularly, an isolated nucleic acid molecule is at least 15 nucleotides in length and hybridizes under stringent conditions to the nucleic acid molecule comprising a nucleotide sequence of herein. In other embodiments, the nucleic acid is at least 30, 50, 100, 250 or more nucleotides in length. Preferably, an isolated nucleic acid homolog comprises a nucleotide sequence which hybridizes under highly stringent conditions to the nucleotide sequence shown herein and restores fertility when expressed in a cytoplasmic male-sterile plant.

As used herein with regard to hybridization, the term "stringent conditions" refers to 6X sodium chloride/sodium citrate (SSC) at about 45°C, followed by one or more washes in 0.2 to 0.5 X SSC, 0.1 to 0.5% SDS at 50 to 68°C. Additionally, the term "highly stringent conditions" refers to 6X SSC at about 45°C, followed by one or more washes in 0.5 X SSC, 0.5% SDS at 68°C. Preferably, an isolated nucleic acid molecule that hybridizes under stringent or highly stringent conditions to a sequence herein corresponds to a naturally occurring nucleic acid molecule. As used herein, a "naturally occurring" nucleic acid molecule refers to an RNA or DNA molecule having a nucleotide sequence that occurs in nature (e.g., encodes a natural protein). Alternatively, the nucleic acid encodes a naturally occurring *Raphanus sativum, Petunia,* or maize protein.

Using the above-described methods, and others known to those of skill in the art, one of ordinary skill in the art can isolate homologs of the fertility restorer nucleic acids proteins. One subset of these homologs comprises allelic variants. As used herein, the term "allelic variant" refers to a nucleotide sequence containing polymorphisms that lead to changes in the amino acid sequences of a fertility restorer protein and that exist within a natural population (e.g., a plant species or variety). Such natural allelic variations can typically result in 1-5% variance in a Fertility restorer nucleic acid. Allelic variants can be identified by sequencing the nucleic acid sequence of interest in a number of different radish, petunia, or maize plants, which can be readily carried out by using hybridization probes to identify the same genetic locus in those plants.

As used herein, the term "analogs" refers to two nucleic acids that have the same or similar function, but that have evolved separately in unrelated organisms. As used herein, the term "orthologs" refers to two nucleic acids from different species, but that have evolved from a common ancestral gene by speciation. Normally, orthologs encode proteins having the same or similar functions. As also used herein, the term "paralogs" refers to two nucleic acids that are related by duplication within a genome. Paralogs usually have different functions, but these functions may be related (Tatusov, R.L. et al. 1997 Science 278(5338):631-637).

Analogs, orthologs, and paralogs of a naturally occurring Fertility restorer nucleic acids can encode proteins that differ from a naturally occurring fertility restorer protein by post-translational modifications, by amino acid sequence differences, or by both. Post-translational modifications include *in vivo* and *in vitro* chemical derivatization of polypeptides, e.g., acetylation, carboxylation, phosphorylation, or glycosylation, and such modifications may occur during polypeptide synthesis or processing or following treatment with isolated modifying enzymes. In particular, orthologs will generally exhibit at least 80-85%, more preferably 90%, and most preferably 95%, 96%, 97%, 98% or even 99% identity or homology with all or part of a naturally occurring fertility restorer amino acid sequence and will exhibit a function similar to a fertility restorer protein. Preferably, a fertility restorer ortholog restores fertility in a male-sterile plant. More preferably, a fertility restorer ortholog restores fertility in a male-sterile *Brassica napus* plant.

In addition to naturally occurring variants of a fertility restorer sequence that may exist in the population, the skilled artisan will further appreciate that changes can be introduced by mutation into a nucleotide sequence shown herein, thereby leading to changes in the amino acid sequence of the encoded fertility restorer protein, without altering the functional activity of the fertility restorer protein. For example, nucleotide substitutions leading to amino acid substitutions at "non-essential" amino acid residues can be made in the sequences. A "non-essential" amino acid residue is a residue that can be altered from the wild-type sequence of one of the fertility restorer proteins without altering the activity of said fertility restorer protein, whereas an "essential" amino acid residue is required for fertility restorer protein activity. Other amino acid residues, however, (e.g., those not within the PPR motif described above) may not be essential for activity and thus are likely to be amenable to alteration without altering fertility restorer activity.

Accordingly, an isolated nucleic acid molecule encoding a fertility restorer protein homologous to a protein sequence herein can be created by introducing one or more nucleotide substitutions, additions or deletions into a nucleotide sequence such that one or more amino acid substitutions, additions or deletions are introduced into the encoded protein. Mutations can be introduced into one of the sequences by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain.

Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a fertility restorer is preferably replaced with another amino acid residue from the same side chain family. Alternatively, in another embodiment, mutations can be introduced randomly along all or part of a fertility restorer coding sequence, such as by saturation mutagenesis, and the resultant mutants can be screened for a fertility restorer activity described herein to identify mutants that retain fertility restorer activity. Following mutagenesis of the sequence, the encoded protein can be expressed and the activity of the protein can be determined by analyzing the viable pollen production of a plant expressing the protein as described above. For example, a fertility restorer mutant that retains activity restores fertility in a male-sterile plant upon its expression in the plant

Additionally, optimized fertility restorer nucleic acids can be created. Preferably, an optimized fertility restorer nucleic acid encodes a fertility restorer that restores fertility in a male-sterile plant, and more particularly male-sterile *Brassica napus* plant. As used herein, "optimized" refers to a nucleic acid that is genetically engineered to increase its expression in a given plant or animal. To provide plant optimized fertility restorer nucleic acids, the DNA sequence of the gene can be modified to 1) comprise codons preferred by highly expressed plant genes; 2) comprise an A+T content in nucleotide base composition to that substantially found in plants; 3) form a plant initiation sequence, 4) eliminate sequences that cause destabilization, inappropriate polyadenylation, degradation and termination of RNA, or that form secondary structure hairpins or RNA splice sites. Increased expression of fertility restorer nucleic acids in plants can be achieved by utilizing the distribution frequency of codon usage in plants in general or a particular plant such as *Brassica napus.*

As used herein, "frequency of preferred codon usage" refers to the preference exhibited by a specific host cell in usage of nucleotide codons to specify a given amino acid. To determine the frequency of usage of a particular codon in a gene, the number of occurrences of that codon in the gene is divided by the total number of occurrences of all codons specifying the same amino acid in the gene. Similarly, the frequency of preferred codon usage exhibited by a host cell can be calculated by averaging the frequency of preferred codon usage in a large number of genes expressed by the host cell. It is preferable that this analysis be limited to genes that are highly expressed by the host cell. The percent deviation of the frequency of preferred codon usage for a synthetic gene from that employed by a host cell is calculated first by determining the percent deviation of the frequency of usage of a single codon from that of the host cell followed by obtaining the average deviation over all codons. As defined herein, this calculation includes unique codons (i.e., ATG and TGG). In general terms, the overall average deviation of the codon usage of an optimized gene from that of a host cell is calculated using the equation 1A = n = 1 Z Xₙ - Yₙ Xₙ times 100 Z where Xₙ = frequency of usage for codon n in the host cell; Yₙ = frequency of usage for codon n in the synthetic gene, n represents an individual codon that specifies an amino acid and the total number of codons is Z. The overall deviation of the frequency of codon usage, A, for all amino acids should preferably be less than about 25%, and more preferably less than about 10%.

Hence, a fertility restorer nucleic acid can be optimized such that its distribution frequency of codon usage deviates, preferably, no more than 25% from that of highly expressed plant genes and, more preferably, no more than about 10%. In addition, consideration is given to the percentage G+C content of the degenerate third base (monocotyledons appear to favor G+C in this position, whereas dicotyledons do not). It is also recognized that the XCG (where X is A, T, C, or G) nucleotide is the least preferred codon in dicots whereas the XTA codon is avoided in both monocots and dicots. Optimized fertility restorer nucleic acids also preferably have CG and TA doublet avoidance indices closely approximating those of the chosen host plant (i.e., *Brassica napus*). More preferably these indices deviate from that of the host by no more than about 10-15%.

In addition to the nucleic acid molecules encoding the fertility restorer proteins described above, another aspect pertains to isolated nucleic acid molecules that are antisense thereto. An "antisense" nucleic acid comprises a nucleotide sequence that is complementary to a "sense" nucleic acid encoding a protein, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire fertility restorer coding strand, or to only a portion thereof. An antisense nucleic acid molecule is antisense to a "coding region" of the coding strand of a nucleotide sequence encoding a fertility restorer protein. The term "coding region" refers to the region of the nucleotide sequence comprising codons that are translated into amino acid residues. In another embodiment, the antisense nucleic acid molecule is antisense to a "noncoding region" of the coding strand of a nucleotide sequence encoding a fertility restorer protein. The term "noncoding region" refers to 5' and 3' sequences that flank the coding region that are not translated into amino acids (i.e., also referred to as 5' and 3' untranslated regions).

An isolated nucleic acid molecule comprises a nucleic acid molecule which is a complement of the nucleotide sequence shown herein, or a portion thereof. A nucleic acid molecule that is complementary to the nucleotide sequence shown herein is one which is sufficiently complementary to the nucleotide sequence shown such that it can hybridize to the nucleotide sequence shown, thereby forming a stable duplex.

Given the coding strand sequences encoding the fertility restorer proteins disclosed herein, antisense nucleic acids of the invention can be designed according to the rules of Watson and Crick base pairing. The antisense nucleic acid molecule can be complementary to the entire coding region of fertility restorer mRNA, but more preferably is an oligonucleotide which is antisense to only a portion of the coding or noncoding region of fertility restorer mRNA. For example, the antisense oligonucleotide can be complementary to the region surrounding the translation start site of fertility restorer mRNA. An antisense oligonucleotide can be, for example, about 5, 10, 15, 20, 25, 30, 35, 40, 45 or 50 or more nucleotides in length.

An antisense nucleic acid as disclosed herein can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecule as disclosed herein are typically administered to a cell or generated *in situ* such that they hybridize with or bind to cellular mRNA and/or genomic DNA encoding a fertility restorer protein to thereby inhibit expression of the protein, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic (including plant) promoter are preferred.

For example, the antisense nucleic acid molecule as disclosed herein is an α-anomeric nucleic acid molecule. An α-anomeric nucleic acid molecule forms specific double-stranded hybrids with complementary RNA in which, contrary to the usual β-units, the strands run parallel to each other (Gaultier et al., 1987 Nucleic Acids. Res. 15:6625-6641). The antisense nucleic acid molecule can also comprise a 2'-o-methylribonucleotide (Inoue et al., 1987 Nucleic Acids Res. 15:6131-6148) or a chimeric RNA-DNA analogue (Inoue et al., 1987 FEBS Lett. 215:327-330).

Alternatively, an antisense nucleic acid is a ribozyme. Ribozymes are catalytic RNA molecules with ribonuclease activity that are capable of cleaving a single-stranded nucleic acid, such as an mRNA, to which they have a complementary region. Thus, ribozymes (e.g., hammerhead ribozymes described in Haselhoff and Gerlach, 1988 Nature 334:585-591) can be used to catalytically cleave fertility restorer mRNA transcripts to thereby inhibit translation of fertility restorer mRNA. A ribozyme having specificity for a fertility restorer -encoding nucleic acid can be designed based upon the nucleotide sequence of a fertility restorer cDNA, corresponding to an ORF of a fertility restorer nucleic acid provided herein or on the basis of a heterologous sequence to be isolated according to methods taught in this invention. For example, a derivative of a *Tetrahymena* L-19 IVS RNA can be constructed in which the nucleotide sequence of the active site is complementary to the nucleotide sequence to be cleaved in a Fertility restorer-encoding mRNA. See, e.g., Cech et al. U.S. Patent No. 4,987,071 and Cech et al. U.S. Patent No. 5,116,742. Alternatively, Fertility restorer mRNA can be used to select a catalytic RNA having a specific ribonuclease activity from a pool of RNA molecules. See, e.g., Bartel, D. and Szostak, J.W., 1993 Science 261:1411-1418.

Alternatively, fertility restorer gene expression can be inhibited by targeting nucleotide sequences complementary to the regulatory region of a fertility restorer nucleotide sequence (e.g., a Fertility restorer promoter and/or enhancer) to form triple helical structures that prevent transcription of a Fertility restorer gene in target cells. See generally, Helene, C., 1991 Anticancer Drug Des. 6(6):569-84; Helene, C. et al., 1992 Ann. N.Y. Acad. Sci. 660:27-36; and Maher, L.J., 1992 Bioassays 14(12):807-15.

In addition to the fertility restorer nucleic acids and proteins described above, the present disclosure encompasses these nucleic acids and proteins attached to a moiety. These moieties include, but are not limited to, detection moieties, hybridization moieties, purification moieties, delivery moieties, reaction moieties, binding moieties, and the like. A typical group of nucleic acids having moieties attached includes probes and primers. Probes and primers typically comprise a substantially isolated oligonucleotide. The oligonucleotide typically comprises a region of nucleotide sequence that hybridizes under stringent conditions to at least about 12, preferably about 25, more preferably about 40, 50 or 75 consecutive nucleotides of a sense strand of the sequence set forth in the sequences, an anti-sense sequence of the sequence set forth in the sequences, or naturally occurring mutants thereof. Primers based on a nucleotide sequences herein can be used in PCR reactions to clone fertility restorer homologs. Probes based on the fertility restorer nucleotide sequences can be used to detect transcripts or genomic sequences encoding the same or homologous proteins. In preferred embodiments, the probe further comprises a label group such as a radioisotope, a fluorescent compound, an enzyme or an enzyme co-factor. Such probes can be used as a part of a genomic marker test kit for identifying cells which express a fertility restorer nucleic acid, such as by measuring a level of a fertility restorer-encoding nucleic acid, in a sample of cells, e.g., detecting fertility restorer mRNA levels or determining whether a genomic fertility restorer gene has been mutated or deleted.

The disclosure further provides an isolated recombinant expression vector comprising a fertility restorer nucleic acid as described above and a nucleotide sequence of interest, wherein expression of the fertility restorer protein in a host plant results in increased produced of viable pollen. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the disclosure is intended to include such other forms of expression vectors, such as viral vectors (e.g., replication defective retroviruses, adenoviruses and adeno-associated viruses), which serve equivalent functions.

The recombinant expression vectors comprise a nucleic acid in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. As used herein with respect to a recombinant expression vector, "operatively linked" is intended to mean that the nucleotide sequence of interest is linked to the regulatory sequence(s) in a manner which allows for expression of the nucleotide sequence (e.g., in an *in vitro* transcription/ translation system or in a host cell when the vector is introduced into the host cell). The term "regulatory sequence" is intended to include promoters, enhancers, and other expression control elements (e.g., polyadenylation signals). Such regulatory sequences are described, for example, in Goeddel, Gene Expression Technology: Methods in Enzymology 185, Academic Press, San Diego, CA (1990) or see: Gruber and Crosby, in: Methods in Plant Molecular Biology and Biotechnology, eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions. It will be appreciated by those skilled in the art that the design of the expression vector can depend on such factors as the choice of the host cell to be transformed, the level of expression of protein desired, etc. The expression vectors can be introduced into host cells to thereby produce proteins or peptides, including fusion proteins or peptides, encoded by nucleic acids as described herein (e.g., fertility restorer proteins, mutant forms of fertility restorer proteins, fusion proteins, etc.).

The recombinant expression vectors can be designed for expression of fertility restorer proteins in prokaryotic or eukaryotic cells. For example, fertility restorer genes can be expressed in multicellular plant cells (see Schmidt, R. and Willmitzer, L., 1988 High efficiency Agrobacterium tumefaciens-mediated transformation of Arabidopsis thaliana leaf and cotyledon explants, Plant Cell Rep. 583-586); Plant Molecular Biology and Biotechnology, C Press, Boca Raton, Florida, chapter 6/7, S.71-119 (1993); F.F. White, B. Jenes et al., Techniques for Gene Transfer, in: Transgenic Plants, Vol. 1, Engineering and Utilization, eds. Kung und R. Wu, 128-43, Academic Press: 1993; Potrykus, 1991 Annu. Rev. Plant Physiol. Plant Molec. Biol. 42:205-225 and references cited therein); *C. glutamicum,* insect cells (using baculovirus expression vectors), yeast and other fungal cells (see Romanos, M.A. et al., 1992 Foreign gene expression in yeast: a review, Yeast 8:423-488; van den Hondel, C.A.M.J.J. et al., 1991 Heterologous gene expression in filamentous fungi, in: More Gene Manipulations in Fungi, J.W. Bennet & L.L. Lasure, eds., p. 396-428: Academic Press: San Diego; and van den Hondel, C.A.M.J.J. & Punt, P.J., 1991 Gene transfer systems and vector development for filamentous fungi, in: Applied Molecular Genetics of Fungi, Peberdy, J.F. et al., eds., p. 1-28, Cambridge University Press: Cambridge), algae (Falciatore et al., 1999 Marine Biotechnology 1(3):239-251) or mammalian cells. Suitable host cells are discussed further in Goeddel, Gene Expression Technology: Methods in Enzymology 185, Academic Press: San Diego, CA (1990). Alternatively, the recombinant expression vector can be transcribed and translated *in vitro,* for example using T7 promoter regulatory sequences and T7 polymerase.

Expression of proteins in prokaryotes is most often carried out with vectors containing constitutive or inducible promoters directing the expression of either fusion or non-fusion proteins. Fusion vectors add a number of amino acids to a protein encoded therein, usually to the amino terminus of the recombinant protein but also to the C-terminus or fused within suitable regions in the proteins. Such fusion vectors typically serve three purposes: 1) to increase expression of a recombinant protein; 2) to increase the solubility of a recombinant protein; and 3) to aid in the purification of a recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D.B. and Johnson, K.S., 1988 Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. For example, the coding sequence of the fertility restorer is cloned into a pGEX expression vector to create a vector encoding a fusion protein comprising, from the N-terminus to the C-terminus, GST-thrombin cleavage site-X protein. The fusion protein can be purified by affinity chromatography using glutathione-agarose resin. Recombinant fertility restorer protein unfused to GST can be recovered by cleavage of the fusion protein with thrombin.

Examples of suitable inducible non-fusion *E. coli* expression vectors include pTrc (Amann et al., 1988 Gene 69:301-315) and pET 11d (Studier et al., Gene Expression Technology: Methods in Enzymology 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a co-expressed viral RNA polymerase (T7 gnl). This viral polymerase is supplied by host strains BL21(DE3) or HMS174(DE3) from a resident λ prophage harboring a T7 gn1 gene under the transcriptional control of the lacUV 5 promoter.

For example, the fertility restorer expression vector is a yeast expression vector. Examples of vectors for expression in yeast S. *cerevisiae* include pYepSec1 (Baldari, et al., 1987 EMBO J. 6:229-234), pMFa (Kurjan and Herskowitz, 1982 Cell 30:933-943), pJRY88 (Schultz et al., 1987 Gene 54:113-123), and pYES2 (Invitrogen Corporation, San Diego, CA). Vectors and methods for the construction of vectors appropriate for use in other fungi, such as the filamentous fungi, include those detailed in: van den Hondel, C.A.M.J.J. & Punt, P.J. (1991) "Gene transfer systems and vector development for filamentous fungi", in: Applied Molecular Genetics of Fungi, J.F. Peberdy, et al., eds., p. 1-28, Cambridge University Press: Cambridge.

Alternatively, the fertility restorer as disclosed herein can be expressed in insect cells using baculovirus expression vectors. Baculovirus vectors available for expression of proteins in cultured insect cells (e.g., Sf 9 cells) include the pAc series (Smith et al., 1983 Mol. Cell Biol. 3:2156-2165) and the pVL series (Lucklow and Summers, 1989 Virology 170:31-39).

Alternatively, the fertility restorer of the invention may be expressed in unicellular plant cells (such as algae) (see Falciatore et al., 1999 Marine Biotechnology 1(3):239-251 and references therein), and more preferably, plant cells from higher plants (e.g., the spermatophytes, such as crop plants). Examples of plant expression vectors include those detailed in: Becker, D., Kemper, E., Schell, J. and Masterson, R., 1992 New plant binary vectors with selectable markers located proximal to the left border, Plant Mol. Biol. 20: 1195-1197; and Bevan, M.W., 1984 Binary Agrobacterium vectors for plant transformation, Nucl. Acid. Res. 12:8711-8721; Vectors for Gene Transfer in Higher Plants; in: Transgenic Plants, Vol. 1, Engineering and Utilization, eds.: Kung and R. Wu, Academic Press, 1993, S. 15-38.

A plant expression cassette preferably contains regulatory sequences capable of driving gene expression in plant cells and operably linked so that each sequence can fulfill its function, for example, termination of transcription by polyadenylation signals. Preferred polyadenylation signals are those originating from *Agrobacterium tumefaciens* t-DNA such as the gene 3 known as octopine synthase of the Ti-plasmid pTiACH5 (Gielen et al., 1984 EMBO J. 3:835) or functional equivalents thereof but also all other terminators functionally active in plants are suitable. As plant gene expression is very often not limited on transcriptional levels, a plant expression cassette preferably contains other operably linked sequences like translational enhancers such as the overdrive-sequence containing the 5'-untranslated leader sequence from tobacco mosaic virus enhancing the protein per RNA ratio (Gallie et al., 1987 Nucl. Acids Research 15:8693-8711).

Plant gene expression must be operatively linked to an appropriate promoter in order to confer gene expression in a timely, cell or tissue specific manner. Preferred are promoters driving constitutive expression (Benfey et al., 1989 EMBO J. 8:2195-2202) like those derived from plant viruses like the 35S CAMV (Franck et al., 1980 Cell 21:285-294), the 19S CaMV (see also U.S. Patent No. 5,352,605 and PCT Application No. WO 8402913) or plant promoters like those from Rubisco small subunit described in U.S. Patent No. 4,962,028.
Especially preferred are those promoters that confer gene expression in specific plant tissues and organs, such as stamens and anthers. In this regard, a promoter which expresses during stamen development would be preferred as such a promoter is particularly appropriate to drive fertility restorer expression resulting in altered pollen production as desired. Examples of such promoters include the AP3 promoter,the Lat52 promoter (Twell, D. et al.,1989, Mol. Gen. Genet. 217, 240-248; Twell, D. et al., 1990, Development 109, 705-715.), the A9 promoter (Paul, W. et al., 1992, Plant Mol. Biol. 19, 611-622.), the fbp1 promoter (Angenent, G.C., 1993,. Plant J. 4, 101-112), the EPF2-5 promoter (Takatsuji, H. et al., 1994, Plant Cell 6, 947-958), and the pfn4 promoter (Christensen, H.E. et al., 1996, Plant J. 10, 269-279). However, the utility of the present methods are not restricted with respect to the promoter. As will be appreciated by one of skill in the art, constitutive promoters and promoters which express during other stages of plant development, for example prior to stamen development, may also be useful in the present methods.

Plant gene expression can also be facilitated via an inducible promoter (for review, see Gatz, 1997 Annu. Rev. Plant Physiol. Plant Mol. Biol. 48:89-108). Chemically inducible promoters are especially suitable if gene expression is wanted to occur in a time specific manner. Examples of such promoters are a salicylic acid inducible promoter (PCT Application No. WO 95/19443), a tetracycline inducible promoter (Gatz et al., 1992 Plant J. 2:397-404) and an ethanol inducible promoter (PCT Application No. WO 93/21334).

Other preferred sequences for use in plant gene expression cassettes are targeting-sequences necessary to direct the gene product in its appropriate cell compartment (for review see Kermode, 1996 Crit. Rev. Plant Sci. 15(4):285-423 and references cited therein) such as the vacuole, the nucleus, all types of plastids like amyloplasts, chloroplasts, chromoplasts, the extracellular space, mitochondria, the endoplasmic reticulum, oil bodies, peroxisomes, and other compartments of plant cells.

Another aspect of the disclosure pertains to host cells into which a recombinant expression vector of the invention has been introduced. The terms "host cell" and "recombinant host cell" are used interchangeably herein. It is understood that such terms refer not only to the particular subject cell but they also apply to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any plant cell. For example, the host cell is a *Brassica napus* plant cell, and most preferably, a stamen or anther cell.

Vector DNA can be introduced into prokaryotic or eukaryotic cells via conventional transformation or transfection techniques. As used herein, the terms "transformation", "transfection", "conjugation" and "transduction" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, natural competence, chemical-mediated transfer and electroporation. Suitable methods for transforming or transfecting host cells including plant cells can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989) and other laboratory manuals such as Methods in Molecular Biology, 1995, Vol. 44, Agrobacterium protocols, ed: Gartland and Davey, Humana Press, Totowa, New Jersey.

In particular, the disclosure provides a method of producing a transgenic plant with a fertility restorer coding nucleic acid and a nucleotide sequence of interest, wherein the plant is a cytoplasmic male-sterile plant and wherein expression of the nucleic acid in the plant results in restoration of fertility of the plant comprising: (a) transforming a plant cell with an expression vector comprising a fertility restorer nucleic acid and a nucleotide sequence of interest, and (b) generating from the plant cell a transgenic plant. The plant cell includes, but is not limited to, a gamete producing cell, a protoplast and any other cell that regenerates into a whole plant.

For such plant transformation, binary vectors such as pBinAR can be used (Höfgen and Willmitzer, 1990 Plant Science 66:221-230). Construction of the binary vectors can be performed by ligation of the cDNA in sense or antisense orientation into the T-DNA. 5-prime to the cDNA a plant promoter activates transcription of the cDNA. A polyadenylation sequence is located 3-prime to the cDNA. Tissue-specific expression can be achieved by using a tissue specific promoter. For constitutive expression within the whole plant, the CaMV 35S promoter can be used. The expressed protein can be targeted to a cellular compartment using a signal peptide, for example for plastids, mitochondria or endoplasmic reticulum (Kermode, 1996 Crit. Rev. Plant Sci. 4 (15):285-423). The signal peptide is cloned 5-prime in frame to the cDNA to archive subcellular localization of the fusion protein. One skilled in the art will recognize that the promoter used should be operatively linked to the nucleic acid such that the promoter causes transcription of the nucleic acid and results in the synthesis of a mRNA which encodes a polypeptide. Alternatively, the RNA can be an antisense RNA for use in affecting subsequent expression of the same or another gene or genes.

Alternate methods of transfection include the direct transfer of DNA into developing flowers via electroporation or *Agrobacterium* mediated gene transfer. *Agrobacterium* mediated plant transformation can be performed using for example the GV3101(pMP90) (Koncz and Schell, 1986 Mol. Gen. Genet. 204:383-396) or LBA4404 (Clontech) *Agrobacterium tumefaciens* strain. Transformation can be performed by standard transformation and regeneration techniques (Deblaere et al., 1994 Nucl. Acids. Res. 13:4777-4788; Gelvin, Stanton B. and Schilperoort, Robert A, Plant Molecular Biology Manual, 2nd Ed. - Dordrecht: Kluwer Academic Publ., 1995. - in Sect., Ringbuc Zentrale Signatur: BT11-P ISBN 0-7923-2731-4; Glick, Bernard R.; Thompson, John E., Methods in Plant Molecular Biology and Biotechnology, Boca Raton : CRC Press, 1993 360 S., ISBN 0-8493-5164-2).

The nucleic acid molecules, proteins, protein homologs, fusion proteins, primers, vectors, and host cells described herein can be used in one or more of the following methods: producing a hybrid plant that contains the nucleic acid molecules as disclosed herein, modulating fertility in a plant, increasing production of viable pollen in a cytoplasmic male-sterile plant, and restoring fertility in a male-sterile plant.

Accordingly, the present disclosure provides a method of producing a hybrid plant, comprising crossing a male-fertile plant containing the Fertility restorer nucleic acids of the present invention with a male-sterile plant, collecting hybrid seed from the male-sterile plant and regenerating the hybrid plant from the seed. For example, the male-sterile plant contains an *ogu* cytoplasmic male sterility determinant. The present disclosure also provides a method of modulating the fertility of a transgenic plant that includes expressing a Fertility restorer nucleic acid in the plant. Preferably, the plant comprises a male-sterility determinant and expression of the nucleic acid sequence in the plant results in increased production of viable pollen by the plant. Preferably, expression of the nucleic acid sequence in the plant results in restoration of fertility of the plant. More preferably, the plant contains an *ogu* cytoplasmic male sterility determinant. The present invention describes using the expression of fertility restorer proteins of *Raphanus sativum,* Petunia, and maize to restore fertility in male-sterile plants. The disclosure also provides a transgenic plant containing a Fertility restorer nucleic acid or a fragment thereof, wherein the plant has increased fertility or viable pollen production as compared to a cytoplasmic male-sterile plant of the same variety. The transgenic plant can be a monocot or a dicot. The disclosure further provides that the transgenic plant can be selected from maize, wheat, rye, oat, triticale, rice, barley, soybean, peanut, rapeseed, canola, pepper, sunflower, Vicia species, pea, alfalfa, bushy plants (coffee, cacao, tea), Salix species, radish, sorghum, pearl millet, cotton, and tobacco. Preferably, the transgenic plant is a canola plant selected from the group consisting of *Brassica napus, Brassica rapa* (or *canipestris), Brassica oleracea, Brassica nigra, Brassica juncea, Sinapis alba,* and *Brassica carinata.* More preferably, the male-sterile plant is *Brassica napus.*

The present disclosure also allows for the production of a true breeding variety of plants that are capable of restoring male fertility in a F1 hybrid descendant of a plant and a male-sterile plant. This type of true breeding variety of a fertility restorer plant is also termed a "restorer line". The terms "variety" and "line" refer to a group of plants within a species that share constant characters that separate them from the typical form and from other possible varieties within that species. While possessing at least one distinctive trait, a variety or line is also characterized by some variation between individuals within the variety or line, based primarily on the Mendelian segregation of traits among the progeny of succeeding generations. A variety or line is considered "true breeding" for a particular trait if it is genetically homozygous for that trait to the extent that, when the true-breeding variety or line is self-pollinated, a significant amount of independent segregation of the trait among the progeny is not observed. In the present disclosure, the trait arises from the transgenic expression of a single DNA sequence introduced into a plant variety or plant line.

Throughout this specification, various publications are referenced.

### EXAMPLES

### Example 1

### Construction of Transformation Vectors

Transformation vectors are constructed as described below. First, a transformation vector is constructed comprising a fertility restorer nucleic acid, driven by a constitutive promoter, and the nucleotide sequence of interest, driven by a desirable promoter. In this vector, the choice of promoter for the fertility restorer gene can also be a male gamete-specific promoter, such as a tapetum-specific promoter. The choice of the fertility restorer gene is determined by the corresponding male sterile gene on the targeted plant for transformation. The fertility restorer gene should be selected based on its ability to compensate for the male sterile phenotype caused by the male sterility gene residing either in the cytoplasm or nucleus of the targeted plant for transformation.

The second vector construct comprises a chimeric DNA molecule (DNA-1) that has a pollen-specific promoter upstream from a male sterility gene, and two unique sequences recognized by the recombinase on both sides of the DNA molecule. An example is a T-DNA containing a male sterility gene driven by tapetum-specific promoter and two unique recombinase sequences within the left and right border sequences. The male sterile gene can be, as is known to those skilled in the art, any gene that causes cell disfunction or cell death.

Another vector is comprised of a recombinase gene flanked by the nucleotide sequence of interest. There are two recombinase recognizing sequences right outside the recombinase gene. This vector is used to transform a plant containing the DNA-1 sequence described above.

For the convenience of demonstration of the selection mechanism, the nucleotide sequence of interest can be replaced by a reporter gene such as a gus (β-glucoronidase) or GFP (green fluorescence protein) gene. The transformation vectors may vary accordingly, as known to those skilled in the art.

### Example 2

### Transformation of cytoplasmic male sterile plants with a restorer gene

A cytoplasmic male sterile line of canola is used for this transformation experiment. Several transformation procedures are tested for demonstration of the selection efficiency, including, but not limited to, Agrobacterium-mediated transformation, particle bombardment, and vacuum-assisted *in planta* transformation. Petioles, hypocotols, apical meristems, axillary meristems, whole seedlings, and flower organs can all be used as the target tissue for transformation. Following DNA delivery to cultured cells, tissue, or seedling, the recovered plant is grown in a controlled environment with steady light and temperature control. This is especially important when cytoplasmic male sterile Brassica plants, such as polima and Ogura are used for transformation due to their temperature sensitivity.

Canola (Brassica napus) male sterile seeds are obtained by crossing a male sterile line with a maintainer line (male fertile, but without the restorer gene), as known to one skilled in the art. Seeds are surface-sterilized and germinated on MS medium containing 3% sucrose and 0.7% phytagar at 25°C, 16 hours light/8 hours dark. Cotyledon petiole and hypocotol explants are ready for transformation after 5 days of germination. Nodal explants are prepared after 35-45 days of germination. Three or more nodes proximal to the apical meristem from each plant are excised; leaves are removed; and nodes are cut into 2-5 mm segments. Each axillary node is separated by cutting the plant stem and hypocotyl longitudinally between the two nodes to produce two nodal explants with one nodal bud for each explant. The excised node explant is then grown in a Magenta box containing MS medium (Murashige and Skoog, 1962) with 3% sucrose to encourage shoot and root formation. A shoot starts to be visible 3-4 days after excision and root formation occurs subsequently. Seedlings recovered from nodal culture appear to be morphologically normal and fertile after transplanted into soil and grown in the greenhouse.

Cotyledon, or hypocotol, or nodal explants, are inoculated with an Agrobacterium strain harboring a plasmid, consisting of a restorer gene flanked with the nucleotide sequence of interest, the GFP gene, as an example. Explants are infected with Agrobacterium by incubation of explants in a bacterial suspension for a short of period of time (5-10 min). The inoculated explants are then cultured on MS medium, 3% sucrose without any selection agent for 2 days. After co-cultivation, the explants are transferred to fresh medium containing carbenicillin to control bacterial growth. A subculture is performed on the same selection medium every 3-4 weeks until root formation. Putative transgenic seedlings are transferred to soil, and grown to maturity for seed production. Similarly, explants prepared as described above are bombarded with gold microparticles coated with DNA molecules that contain the fertility restorer nucleic acid and the nucleotide sequence of interest. The recovered seedlings are grown in a controlled environment chamber for seed production.

*In planta* transformation is also performed in seedlings before flowering stage according to Trieu et al., 2000. Agrobacterium strain containing a DNA construct with the fertility restorer nucleic acid and the nucleotide sequence of interest is used for the vacuum infiltration of the seedling. Seeds are harvested from the mature plants grown in a growth chamber.

Pollen formed on a male sterile plant after transformation with the fertility restorer nucleic acid construct is collected and examined using PCR, Southern blot analyses, and visualization under a fluorescence microscope for detection of GFP. PCR and Southern blot analyses are performed on those putative primary transgenic seedlings to confirm integration of the T-DNA, specifically the presence of the nucleotide sequence of interest, into the plant genome.

### Example 3

### Production of Male Sterile Lines and Restoration of Male Sterility in These Lines

A canola cultivar is transformed with a vector comprising a male sterility nucleic acid flanked by particular sequences recognized by a site specific recombinase. Examples of genes that cause cytoplasmic or nuclear male sterility when expressed in a plant have been described in the literature and are known to those of ordinary skill in the art. For example, Mariani et al. have described that the introduction of an RNAse (barnase) under the control of a tapetum-specific promoter into the nuclear genome results in nuclear male sterility (Mariani et al., 1990, Nature 347: 737; EP 0,344,029). International Patent Application WO 89/10396 ) discloses that certain DNAses, RNAses, proteases, and enzymes involved in phytohormone synthesis may result in male sterility. International Patent Application WO 90/08831 discloses that inhibition of cell-respiration by expression of a disrupted gene such as an ATPase, also results in male sterility in the plant. Finally, International Patent Application WO 90/08830 teaches that expression of a protein inhibitor gene, or "killer gene," in male flowers causing death of the anthers.

Each of the genes described above (and others described in the literature) that result in male sterility in a plant in which the gene is expressed may be used in practicing the present invention. Accordingly, the male sterility nucleic acid is introduced into a plasmid comprising a heterologous promoter, and the promoter and male sterility nucleic acid are flanked by recombinase sites. These plasmids are used to transform male fertile plants or plant cells, and the resulting male sterile plants or plant cells are suitable for further transformation with a second plasmid construct.

The second plasmid construct comprises a nucleic acid encoding a recombinase under the control of a heterologous promoter and a nucleotide sequence of interest. Upon transformation of the male sterile plants described above, transgenic plants (i.e. plants comprising the nucleotide sequence of interest) are identified as those plants that form seeds by self-pollination. Transgenic plants express the recombinase nucleic acid, and the recombinase interacts with the recombinase sites flanking the male sterility nucleic acid, causing excision of the male sterility nucleic acid and thereby restoring male fertility in the transgenic plant. Because the nucleotide of interest is closely linked to the recombinase nucleic acid on this second plasmid construct, the restored capability of the plant to form seeds by self-pollination indicates the presence of the other transgene, i.e. the nucleotide sequence of interest.

### Example 4

### Segregation of the gene of interest from CMS background

Transgenic seeds collected from the primary (T₀) transgenic plants with the cytoplasmic male sterile background are germinated in the soil. PCR and Southern blot hybridization analyses are performed to confirm the integration and the copy numbers of the fertility restorer nucleic acid and the nucleotide sequence of interest. A wild-type line of the same species, e.g. Brassica, and the transgenic line are grown in the greenhouse as the female and male parents, respectively, for the hybridization. When both the wild-type and transgenic parent lines reach the flowering stage, the wild-type female parent is carefully emasculated, and the pollen from the transgenic plant is used to pollinate the wild-type female parent. The flower organs after pollination are covered with a paper bag to prevent further pollination from other pollen sources.

The hybrid seeds are again harvested from the cross-pollinated flower, and germinated in the soil. Analyses of the seedlings is performed using PCR and Southern hybridization to confirm the presence of the transgenes (e.g. the fertility restorer nucleic acid and the nucleotide sequence of interest), and also the male sterility nucleic acid as well. The results show the absence of the male sterility nucleic acid, and the presence of the transgenes. Because the transgenic plant is used as the male parent, and the male sterility is cytoplasmic, the hybrid progeny contains no CMS background.

In a variation, the homozygous transgenic lines are produced before removing the CMS background. The seeds (T1) collected from the primary (T0) transgenic plants are grown in a greenhouse to maturity, and the plants are selfed to produce T2 seeds. Segregation of male sterility is observed in terms of the pollen development, and the presence of the transgenes among the T1 population, using PCR and Southern hybridization. Subsequently, the T2 generation of plants are grown to maturity in a greenhouse. Segregation analysis is also performed to select the progeny containing the transgene. Those PCR-positive plants produce viable seeds in a greenhouse, while PCR-negative plants remain sterile. Segregation experiment continues until no segregation is observed. Plants identified, therefore, are homozygous for the allele with the transgene, and are used as male parent for generation hybrid plants with a wild-type female parent to remove the CMS background, outlined above.

### Example 5

### Segregation of the gene of interest from the nuclear male sterility background

In this example, a segregation experiment is conducted to separate the transgenes (the fertility restorer nucleic acid and the nucleotide sequence of interest) from the nuclear male sterility nucleic acid in a transgenic plant generated in Example 2.

A similar approach is taken, as outlined in the Example 4 for producing a line that is homozygous for the transgene. The segregation is determined by two methods, (1) the sterility of the selfed progeny, and (2) detection of the transgene and the nuclear male sterility gene. The plant containing only the transgene is selected, and therefore, the nuclear male sterility gene is removed from this progeny.

### Example 6

### Engineering Transgenic Brassica plants with Superior Qualities

A person skilled in the art could use the Brassica plant of this invention to develop a Brassica plant which is a restorer of fertility for ogura cytoplasmic male sterility, produces oilseeds having low glucosinolate content, and which is resistant to the family of imidazoline herbicides, sold by Cyanamid under trademarks such as PURSUIT. Resistance to imidazoline herbicides is conferred by the gene AHAS or ALS. One skilled in the art could introduce the mutant form of AHAS present in varieties such as the Pioneer® spring canola variety, 45A71, into a Brassica plant which also carries the Rf gene for the ogura cytoplasm. Alternatively, one could introduce a modified form of the AHAS gene with a suitable promoter into a canola plant cell through any of several methods. Basically, the genes are introduced into a plant cell, such as a plant cell of this invention carrying the fertility restorer nucleic acid for ogura cytoplasmic male sterility, and then the plant cell is grown into a Brassica plant.

### APPENDIX

*cDNA sequence of Flp recombinase* (SEQ ID NO:1)

1 atgccacaat ttggtatatt atgtaaaaca ccacctaagg tgcttgttcg tcagtttgtg
61 gaaaggtttg aaagaccttc aggtgagaaa atagcattat gtgctgctga actaacctat
121 ttatgttgga tgattacaca taacggaaca gcaatcaaga gagccacatt catgagctat
181 aatactatca taagcaattc gctgagtttc gatattgtca ataaatcact ccagtttaaa
241 tacaagacgc aaaaagcaac aattctggaa gcctcattaa agaaattgat tcctgcttgg
301 gaatttacaa ttattcctta ctatggacaa aaacatcaat ctgatatcac tgatattgta
361 agtagtttgc aattacagtt cgaatcatcg gaagaagcag ataagggaaa tagccacagt
421 aaaaaaatgc ttaaagcact tctaagtgag ggtgaaagca tctgggagat cactgagaaa
481 atactaaatt cgtttgagta tacttcgaga tttacaaaaa caaaaacttt ataccaattc
541 ctcttcctag ctactttcat caattgtgga agattcagcg atattaagaa cgttgatccg
601 aaatcattta aattagtcca aaataagtat ctgggagtaa taatccagtg tttagtgaca
661 gagacaaaga caagcgttag taggcacata tacttcttta gcgcaagggg taggatcgat
721 ccacttgtat atttggatga atttttgagg aattctgaac cagtcctaaa acgagtaaat
781 aggaccggca attcttcaag caataaacag gaataccaat tattaaaaga taacttagtc
841 agatcgtaca ataaagcttt gaagaaaaat gcgccttatt caatctttgc tataaaaaat
901 ggcccaaaat ctcacattgg aagacatttg atgacctcat ttctttcaat gaagggccta
961 acggagttga ctaatgttgt gggaaattgg agcgataagc gtgcttctgc cgtggccagg
1021 acaacgtata ctcatcagat aacagcaata cctgatcact acttcgcact agtttctcgg
1081 tactatgcat atgatccaat atcaaaggaa atgatagcat tgaaggatga gactaatcca
1141 attgaggagt ggcagcatat agaacagcta aagggtagtg ctgaaggaag catacgatac
1201 cccgcatgga atgggataat atcacaggag gtactagact acctttcatc ctacataaat
1261 agacgcatat aa

*cDNA sequence for Cre recombinase* (SEQ ID NO:2)

1 tgcgcagctg gacgtaaact cctcttcaga cctaataact tcgtatagca tacattatac
61 gaagttatat taagggttat tgaatatgat caatttacct gtaaatccat acagttcaat
121 accttagcag gtcaaatagt gaccacttga tcatttgatc aaggttgcgc tacgtaaaat
181 ctgtgaaaaa ttggcggtgt tagtcctaca gatttcgcgt accacttagc accaccaatc
241 aatcagaggt gaaaaatggg atattcaact gctaaagtgt ccactcatct tgagcttgag
301 aaaaaccgtg gttactggcg ggcaaaaggg tttgatcgtg atagttgcca actgtcatta
361 tcgcgcggtg aagagaaaat agaacgcacg cgcggtcgct ggcgtttcta tgacgagaac
421 cataaacagg taaaggcaga gccgatcctg tacactttac ttaaaaccat tatctgagtg
481 ttaaatgtcc aatttactga ccgtacacca aaatttgcct gcattaccgg tcgatgcaac
541 gagtgatgag gttcgcaaga acctgatgga catgttcagg gatcgccagg cgttttctga
601 gcatacctgg aaaatgcttc tgtccgtttg ccggtcgtgg gcggcatggt gcaagttgaa
661 taaccggaaa tggtttcccg cagaacctga agatgttcgc gattatcttc tatatcttca
721 ggcgcgcggt ctggcagtaa aaactatcca gcaacatttg ggccagctaa acatgcttca
781 tcgtcggtcc gggctgccac gaccaagtga cagcaatgct gtttcactgg ttatgcggcg
841 gatccgaaaa gaaaacgttg atgccggtga acgtgcaaaa caggctctag cgttcgaacg
901 cactgatttc gaccaggttc gttcactcat ggaaaatagc gatcgctgcc aggatatacg
961 taatctggca tttctgggga ttgcttataa caccctgtta cgtatagccg aaattgccag
1021 gatcagggtt aaagatatct cacgtactga cggtgggaga atgttaatcc atattggcag
1081 aacgaaaacg ctggttagca ccgcaggtgt agagaaggca cttagcctgg gggtaactaa
1141 actggtcgag cgatggattt ccgtctctgg tgtagctgat gatccgaata actacctgtt
1201 ttgccgggtc agaaaaaatg gtgttgccgc gccatctgcc accagccagc tatcaactcg
1261 cgccctggaa gggatttttg aagcaactca tcgattgatt tacggcgcta aggatgactc
1321 tggtcagaga tacctggcct ggtctggaca cagtgcccgt gtcggagccg cgcgagatat
1381 ggcccgcgct ggagtttcaa taccggagat catgcaagct ggtggctgga ccaatgtaaa
1441 tattgtcatg aactatatcc gtaacctgga tagtgaaaca ggggcaatgg tgcgcctgct
1501 ggaagatggc gattagccat taacgcgtaa atgattgcta taattagttg ata

*cDNA sequence of φC31 recombinase* (SEQ ID NO:3)

1 gtggacacgt acgcgggtgc ttacgaccgt cagtcgcgcg agcgcgagaa ttcgagcgca
61 gcaagcccag cgacacagcg tagcgccaac gaagacaagg cggccgacct tcagcgcgaa

121 gtcgagcgcg acgggggccg gttcaggttc gtcgggcatt tcagcgaagc gccgggcacg
181 tcggcgttcg ggacggcgga gcgcccggag ttcgaacgca tcctgaacga atgccgcgcc
241 gggcggctca acatgatcat tgtctatgac gtgtcgcgct tctcgcgcct gaaggtcatg
301 gacgcgattc cgattgtctc ggaattgctc gccctgggcg tgacgattgt ttccactcag
361 gaaggcgtct tccggcaggg aaacgtcatg gacctgattc acctgattat gcggctcgac
421 gcgtcgcaca aagaatcttc gctgaagtcg gcgaagattc tcgacacgaa gaaccttcag
481 cgcgaattgg gcgggtacgt cggcgggaag gcgccttacg gcttcgagct tgtttcggag
541 acgaaggaga tcacgcgcaa cggccgaatg gtcaatgtcg tcatcaacaa gcttgcgcac
601 tcgaccactc cccttaccgg acccttcgag ttcgagcccg acgtaatccg gtggtggtgg
661 cgtgagatca agacgcacaa acaccttccc ttcaagccgg gcagtcaagc cgccattcac
721 ccgggcagca tcacggggct ttgtaagcgc atggacgctg acgccgtgcc gacccggggc
781 gagacgattg ggaagaagac cgcttcaagc gcctgggacc cggcaaccgt tatgcgaatc
841 cttcgggacc cgcgtattgc gggcttcgcc gctgaggtga tctacaagaa gaagccggac
901 ggcacgccga ccacgaagat tgagggttac cgcattcagc gcgacccgat cacgctccgg
961 ccggtcgagc ttgattgcgg accgatcatc gagcccgctg agtggtatga gcttcaggcg
1021 tggttggacg gcagggggcg cggcaagggg ctttcccggg ggcaagccat tctgtccgcc
1081 atggacaagc tgtactgcga gtgtggcgcc gtcatgactt cgaagcgcgg ggaagaatcg
1141 atcaaggact cttaccgctg ccgtcgccgg aaggtggtcg acccgtccgc acctgggcag
1201 cacgaaggca cgtgcaacgt cagcatggcg gcactcgaca agttcgttgc ggaacgcatc
1261 ttcaacaaga tcaggcacgc cgaaggcgac gaagagacgt tggcgcttct gtgggaagcc
1321 gcccgacgct tcggcaagct cactgaggcg cctgagaaga gcggcgaacg ggcgaacctt
1381 gttgcggagc gcgccgacgc cctgaacgcc cttgaagagc tgtacgaaga ccgcgcggca
1441 ggcgcgtacg acggacccgt tggcaggaag cacttccgga agcaacaggc agcgctgacg
1501 ctccggcagc aaggggcgga agagcggctt gccgaacttg aagccgccga agccccgaag
1561 cttccccttg accaatggtt ccccgaagac gccgacgctg acccgaccgg ccctaagtcg
1621 tggtgggggc gcgcgtcagt agacgacaag cgcgtgttcg tcgggctctt cgtagacaag
1681 atcgttgtca cgaagtcgac tacgggcagg gggcagggaa cgcccatcga gaagcgcgct
1741 tcgatcacgt gggcgaagcc gccgaccgac gacgacgaag acgacgccca ggacggcacg
1801 gaagacgtag cggcgtag

*cDNA sequence of R recombinase* (SEQ ID NO:4)

1 atgcaattga ccaaggatac tgaaatttca acaattaata gacaaatgtc agacttcagc
61 gagcttagtc aaatccttcc cttacaccag atctctaaaa taaaagacat tttggagaat
121 gagaaccctt tacctaagga aaaactagcc tcccatctga cgatgataat tttgatggcc
181 aatttggcga gtcagaaaag aaaggacgtt cctgtcaagc gctccacttt cttgaaatac
241 cagaggtcta tttcaaaaac attgcaatac gattcttcca ctaaaacggt atcctttgaa
301 taccatttga aagacccaag taagctaata aaaggactcg aagatgttgt atcgccctat
361 agatttgttg tgggggtaca cgaaaagcct gatgatgtaa tgagtcattt atctgctgta
421 catatgagaa aggaagcagg tcggaaaaga gatttgggta acaagattaa tgatgaaatc
481 actaaaatcg ctgaaacaca ggaaactatt tgggggtttg tcggaaagac tatggactta
541 atagaagcta gaactactcg gcctacaaca aaagccgcgt acaatctttt gcttcaggcg
601 acttttatga attgttgtag agcagacgac ttaaagaata ccgatatcaa gacatttgaa
661 gttattccag acaagcacct tggccgcatg ctacgtgctt ttgtcccaga gactaagact
721 ggtactcgtt ttgtctattt cttcccttgt aaaggacgat gcgacccgct gttggcacta
781 gattcatacc tccaatggac agatcctatt cccaaaactc gaactactga tgaggatgca
841 cgctatgatt accagcttct caggaacagt ctgttaggct catatgacgg gtttatctct
901 aaacaatcgg atgagagcat ctttaaaata cccaatggac cgaaagcgca tttaggcagg
961 catgtgacag cttcatatct ttcaaataat gagatggata aagaagccac gctctatggt
1021 aattggtctg ccgcgcggga agaaggtgtt agccgtgtgg ctaaggctcg atacatgcac
1081 acgatagaaa agagtccgcc ttcttattta tttgcgtttc tatcggggtt ttataatatc
1141 acagccgaaa gggcgtgtga gttggttgat ccaaatagca acccttgtga acaagacaag
1201 aatattccta tgatatcaga tatcgagaca ttgatggctc ggtatggaaa gaatgcagaa
1261 ataataccaa tggatgtatt ggtttttttg agcagctatg ccaggttcaa gaataacgaa
1321 ggtaaagagt ataagttaca agcaagaagt tcccgaggcg tcccagattt tccggataat
1381 gggagaactg cactttataa cgcgttaaca gcggcacatg tgaagcgccg aaagattagc
1441 attgtcgtcg gacgatccat cgacacctcc tga

*cDNA sequence of Gin recombinase* (SEQ ID NO:5)

1 gtgctgattg gctatgtaag ggtatcaaca aatgaccaga atacagacct gcaacgaaac
61 gctcttgttt gtgcaggatg tgaacaaata tttgaagata aattaagcgg aacaaggaca

121 gaccgaccgg gattaaaacg cgctttaaag cgccttcaaa aaggtgacac actggttgtc
181 tggaaactgg atcgcctcgg gcgaagcatg aaacatttga tttctctcgt aggggaatta
241 cgagagcgag ggattaattt tcgcagtctt actgacagta ttgatacgtc atctccaatg
301 gggcgttttt tcttccacgt tatgggtgcc ctggctgaaa tggaacgaga actaattatc
361 gagcgaacga tggctggact tgctgccgcc agaaataaag gccgtattgg tgggcgacca
421 cctaaactaa ccaaagcgga atgggagcag gccgggcgtt tattagcaca aggaatcccc
481 cgcaagcagg ttgcattgat ctacgatgtg gccctgtcaa ctctgtataa aaaacacccc
541 gcgaaacgag cgcatataga aaacgacgat cgaatcaatt aa

*cDNA sequence of β-recombinase* (SEQ ID NO:6)

1 atggctaaaa ttggttatgc acgtgtcagt agcaaagaac agaacttaga tcggcaatta
 61 caagcgttac agggcgtttc taaggtcttt tcagacaaat taagcggtca atcggtcgaa
121 cgtccacaat tacaagctat gcttaactat attcgtgaag gggatattgt tgtagttact
181 gaattagatc gattaggacg gaataataaa gaattaacag aattgatgaa tgccattcaa
241 caaaaaggag caacgcttga agttttgaat ttgccctcta tgaatgggat tgaagatgaa
301 aatttaaggc gtttgattaa taatttagtg attgaattat ataagtatca agcggaatca
361 gaacgcaaac gtattaaaga acgacaagcc caagggattg aaatagctaa gtcaaaagga
421 aaattcaaag gtcgtcagca taaatttaaa gaaaatgatc cacgtttaaa acatgctttt
481 gatttatttt tgaacggttg ttccgataaa gaagttgagg aacaaactgg aattaatcgc
541 agaacgttta gaaggtatcg tacaagatac aacgtgacag tcgatcaaag aaaaaacaag
601 ggaaagaggg atagttaa

## Claims

1. A method of producing a transgenic male fertile plant comprising a nucleotide sequence of interest, comprising
a) providing a male sterile plant comprising a male sterility nucleic acid operatively linked to a heterologous promoter flanked by recombinase sites;
b) transforming said male sterile plant with a vector comprising a nucleic acid encoding a recombinase flanked at both ends by recombinase sites right outside the nucleic acid encoding the recombinase and the nucleotide sequence of interest;
c) expressing the nucleic acid encoding the recombinase in the plant; and
d) identifying the transgenic male fertile plant by selecting for the plant that forms seeds by self-pollination.

2. The method of Claim 1, wherein said male sterile plant is Brassica.

3. The method of Claim 1, wherein said male sterile plant is *Brassica napus.*

4. The method of Claim 1, wherein said recombinase is selected from the group consisting of Flp, Cre, ΦC31, R, Gin, and β-recombinase.

5. The method of Claim 1, wherein the heterologous promoter is a pollen-specific promoter.

6. The method of Claim 1, wherein the male sterility nucleic acid comprises a nucleotide sequence encoding a polypeptide selected from the group consisting of barnase, protein inhibitors, DNAses, RNAses, proteases, enzymes involved in phytohormone synthesis, and disrupter proteins.

7. A method of identifying a transformed plant, comprising:
a) providing a vector comprising a nucleic acid encoding a recombinase and the nucleotide sequence of interest, wherein the nucleic acid encoding the recombinase is flanked at both ends by recombination sites;
b) introducing said vector into a plant cell derived from a male sterile plant comprising a male sterility nucleic acid operatively linked to a heterologous promoter flanked by recombinase sites;
c) regenerating a plant from said transformed plant cell; and
d) identifying a plant from step c) that forms seeds by self-pollination, such that said identified plant is transformed.

8. The method of Claim 7, further comprising an earlier step of transforming a male fertile plant with a male sterility nucleic acid operatively linked to a heterologous promoter flanked by recombinase sites to create a male sterile plant.

9. The method of Claim 7, wherein said male sterile plant is Brassica.

10. The method of Claim 7, wherein said male sterile plant is *Brassica napus.*

11. The method of Claim 7, wherein the recombinase is selected from the group consisting of Flp, Cre, ΦC31, R, Gin, and β-recombinase.

12. Use of a vector in the method of Claim 7 comprising a nucleic acid encoding a recombinase and the nucleotide sequence of interest, wherein the nucleic acid encoding the recombinase is flanked at both ends by recombination sites.

13. Use of the expression vector of Claim 12, wherein the recombinase is selected from the group consisting of Flp, Cre, ΦC31, R, Gin, and β-recombinase.

14. Use of the expression vector of Claim 12, wherein the nucleotide sequence of interest is an altered Als nucleic acid that results in a plant's increased tolerance to imidazolinone herbicides when the altered Als nucleic acid is expressed in the plant.

## Patentansprüche

1. Verfahren zur Herstellung einer transgenen männlich fertilen Pflanze, umfassend eine Nukleotidsequenz von Interesse, umfassend
a) Bereitstellen einer männlich sterilen Pflanze, umfassend eine männliche Sterilitätsnukleinsäure, die operativ mit einem heterologen Promotor verbunden ist, flankiert von Rekombinasestellen;
b) Transformieren der männlich sterilen Pflanze mit einem Vektor, umfassend eine Nukleinsäure, die eine Rekombinase und die Nukleotidsequenz von Interesse kodiert, wobei die Nukleinsäure welche die Rekombinase kodiert an beiden Enden von Rekombinasestellen flankiert ist;
c) Exprimieren der Nukleinsäure welche die Rekombinase kodiert in der Pflanze; und
d) Identifizieren der transgenen männlich fertilen Pflanze durch Selektieren nach der Pflanze die durch Selbstbestäubung Samen bildet.

2. Verfahren nach Anspruch 1, wobei die männlich sterile Pflanze Brassica ist.

3. Verfahren nach Anspruch 1, wobei die männlich sterile Pflanze *Brassica napus* ist.

4. Verfahren nach Anspruch 1, wobei die Rekombinase ausgewählt ist aus der Gruppe bestehend aus Flp, Cre, ΦC31, R, Gin und β-Rekombinase.

5. Verfahren nach Anspruch 1, wobei der heterologe Promotor ein pollenspezifischer Promotor ist.

6. Verfahren nach Anspruch 1, wobei die männliche Sterilitätsnukleinsäure eine Nukleotidsequenz umfasst, welche ein Polypeptid kodiert, das ausgewählt ist aus der Gruppe bestehend aus Barnase, Protein-Inhibitoren, DNAsen, RNAsen, Proteasen, Enzymen die an der Phytohormonsynthese beteiligt sind und Unterbrecherproteinen.

7. Verfahren zur Identifizierung einer transformierten Pflanze, umfassend:
a) Bereitstellen eines Vektors, welcher eine Nukleinsäure, die eine Rekombinase kodiert und die Nukleotidsequenz von Interesse umfasst, wobei die Nukleinsäure welche die Rekombinase kodiert an beiden Enden von Rekombinasestellen flankiert ist;
b) Einbringen des Vektors in eine von einer männlich sterilen Pflanze abgeleitete Pflanzenzelle, umfassend eine männliche Sterilitätsnukleinsäure die operativ mit einem heterologen Promotor verbunden ist, flankiert von Rekombinasestellen,
c) Regenerieren einer Pflanze aus der transformierten Pflanzenzelle, und
d) Identifizieren einer Pflanze aus Schritt c) die durch Selbstbestäubung Samen bildet, sodass die identifizierte Pflanzen transformiert ist.

8. Verfahren nach Anspruch 7, ferner umfassend einen früheren Schritt des Transformierens einer männlich fertilen Pflanze mit einer männlichen Sterilitätsnukleinsäure, die operativ mit einem heterologen Promotor verbunden ist, flankiert von Rekombinasestellen, um eine männlich sterile Pflanze zu erzeugen.

9. Verfahren nach Anspruch 7, wobei die männlich sterile Pflanze Brassica ist.

10. Verfahren nach Anspruch 7, wobei die männlich sterile Pflanze *Brassica napus* ist.

11. Verfahren nach Anspruch 7, wobei die Rekombinase ausgewählt ist aus der Gruppe bestehend aus Flp, Cre, ΦC31, R, Gin und β-Rekombinase.

12. Verwendung eines Vektors, welcher eine Nukleinsäure, die eine Rekombinase kodiert und die Nukleotidsequenz von Interesse umfasst, wobei die Nukleinsäure welche die Rekombinase kodiert an beiden Enden von Rekombinasestellen flankiert ist, in dem Verfahren von Anspruch 7.

13. Verwendung des Expressionsvektors von Anspruch 12, wobei die Rekombinase ausgewählt ist aus der Gruppe bestehend aus Flp, Cre, ΦC31, R, Gin und β-Rekombinase.

14. Verwendung des Expressionsvektors von Anspruch 12, wobei die Nukleotidsequenz von Interesse eine veränderte Als Nukleinsäure ist, die zu einer erhöhten Toleranz der Pflanze gegen Imidazolinon-Herbizide führt, wenn die veränderte Als Nukleinsäure in der Pflanze exprimiert wird.

## Revendications

1. Procédé de production d'un plante mâle fertile transgénique comprenant une séquence nucléotidique d'intérêt, comprenant
a) la mise à disposition d'une plante mâle stérile comprenant un acide nucléique de stérilité mâle, lié d'une manière opérationnelle à un promoteur hétérologue flanqué de sites de reconnaissance par la recombinase ;
b) la transformation de ladite plante mâle stérile avec un vecteur comprenant un acide nucléique codant pour une recombinase et la séquence nucléotidique d'intérêt, l'acide nucléique codant pour la recombinase étant flanqué, en ses deux extrémités, par des sites de recombinaison ;
c) l'expression de l'acide nucléique codant pour la recombinase dans la plante ; et
d) l'identification de la plante mâle fertile transgénique par sélection portant sur la plante qui forme des graines par auto-pollinisation.

2. Procédé de la revendication 1, dans lequel ladite plante mâle stérile est Brassica.

3. Procédé de la revendication 1, dans lequel ladite plante mâle stérile est *Brassica napus.*

4. Procédé de la revendication 1, dans lequel ladite recombinase est choisie dans le groupe consistant en les recombinases Flp, Cre, ΦC31, R, Gin et β.

5. Procédé de la revendication 1, dans lequel le promoteur hétérologue est un promoteur spécifique de pollen.

6. Procédé de la revendication 1, dans lequel l'acide nucléique de stérilité mâle comprend une séquence nucléotidique codant pour un polypeptide choisi dans le groupe consistant en la barnase, les inhibiteurs de protéines, les ADN-ases, les ARN-ases, les protéases, les enzymes impliquées dans la synthèse des phytohormones, et les protéines de rupture.

7. Procédé pour l'identification d'une plante transformée, comprenant :
a) la mise à disposition d'un vecteur comprenant un acide nucléique codant pour une recombinase et la séquence nucléotidique d'intérêt, l'acide nucléique codant pour la recombinase étant flanqué, en ses deux extrémités, par des sites de recombinaison ;
b) l'introduction dudit vecteur dans une cellule végétale qui dérive d'une plante mâle stérile comprenant un acide nucléique de stérilité mâle lié d'une manière opérationnelle à un promoteur hétérologue flanqué par des sites de reconnaissance par une recombinase ;
c) la régénération d'une plante à partir de ladite cellule végétale transformée ; et
d) l'identification d'une plante de l'étape c) qui forme des graines par auto-pollinisation, telle que ladite plante identifiée soit transformée.

8. Procédé de la revendication 7, qui comprend en outre une étape préalable de transformation d'une plante mâle stérile avec un acide nucléique de stérilité mâle lié d'une manière opérationnelle à un promoteur hétérologue flanqué par des sites de reconnaissance par une recombinase pour créer une plante mâle stérile.

9. Procédé de la revendication 7, dans lequel ladite plante mâle stérile est Brassica.

10. Procédé de la revendication 7, dans lequel ladite plante mâle stérile est *Brassica napus.*

11. Procédé de la revendication 7, dans lequel la recombinase est choisie dans le groupe consistant en les recombinases Flp, Cre, Φ31, R, Gin et β.

12. Utilisation d'un vecteur comprenant un acide nucléique codant pour une recombinase et la séquence nucléotidique d'intérêt, l'acide nucléique codant pour la recombinase étant flanqué en ses deux extrémités par des sites de recombinaison dans le procédé de la revendication 7.

13. Utilisation du vecteur d'expression de la revendication 12, pour laquelle la recombinase est choisie dans le groupe consistant en les recombinases Flp, Cre, Φ31, R, Gin et β.

14. Utilisation du vecteur d'expression de la revendication 12, pour laquelle la séquence nucléotidique d'intérêt est un acide nucléique Als altéré, qui conduit à une tolérance accrue de la plante aux herbicides de type imidazolinone quand l'acide nucléique Als altéré est exprimé dans la plante.
